(19) Europäisches Patentamt
European Patent Office
Office européen des brevets

(11) Veröffentlichungsnummer: **0 138 034**
**B1**

(12) # EUROPÄISCHE PATENTSCHRIFT

(45) Veröffentlichungstag der Patentschrift: **29.03.89**

(51) Int. Cl.⁴: **C 07 D 471/14**, C 07 D 401/06, A 61 K 31/44

(21) Anmeldenummer: **84110589.3**

(22) Anmeldetag: **06.09.84**

(54) **Substituierte Pyrido(1,2-c)imidazo((1,2-a)benzimidazole, Verfahren zu ihrer Herstellung, ihre Verwendung sowie pharmazeutische Präparate auf Basis dieser Verbindungen.**

(30) Priorität: **15.09.83 DE 3333314**

(43) Veröffentlichungstag der Anmeldung: **24.04.85 Patentblatt 85/17**

(45) Bekanntmachung des Hinweises auf die Patenterteilung: **29.03.89 Patentblatt 89/13**

(84) Benannte Vertragsstaaten: **AT BE CH DE FR GB IT LI LU NL SE**

(56) Entgegenhaltungen:
**DE-A-2 211 796**
**GB-A-2 028 317**
**US-A-4 254 127**

(73) Patentinhaber: **HOECHST AKTIENGESELLSCHAFT, Postfach 80 03 20, D-6230 Frankfurt am Main 80 (DE)**

(72) Erfinder: **Hitzel, Volker, Dr., Kantstrasse 1b, D-6238 Hofheim am Taunus (DE)**
Erfinder: **Rackur, Gerhard, Dr., Gundelhardtstrasse 2, D-6233 Kelkheim (Taunus) (DE)**
Erfinder: **Bickel, Martin, Dr., Mittelstedter Weg 3, D-6380 Bad Homburg (DE)**

EP 0 138 034 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Bei der Ulkustherapie werden neben Antacida und Carbenoxolon auch Sekretionshemmer wie $H_2$-Antihistaminika (z. B. Cimetidin; Brit. J. Pharmacol. 53 [1975] 435 p) oder Parasympatholytika eingesetzt. In der DE-A-2 548 340, der EP-A-5129 und der DE-A-3 240 248 sind ferner substituierte 2-Benzimidazol-2'-pyridylsulfoxide beschrieben, die die exogen und endogen stimulierte Magensäuresekretion hemmen und deshalb in pharmazeutischen Präparaten zur Behandlung von gastrointestinalen Läsionen, insbesondere von Geschwüren des Magens und des Zwölffingerdarms eingesetzt werden sollen.

Es wurde nun überraschenderweise gefunden, daß die beispielsweise durch Cyclisierung bzw. Umlagerung entsprechender Benzimidazol- oder Benzimidazolon-Derivate zugänglichen Pyrido(1,2-c)imidazo(1,2-a)benzimidazole hochwirksame Magensäuresekretionshemmer sind.

Die Erfindung betrifft Verbindungen der Formel I

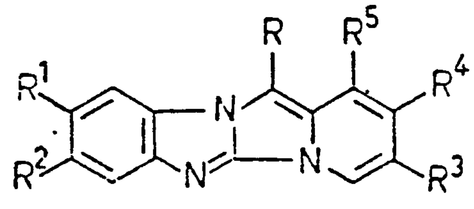

(I)

in welcher

$R^1$ und $R^2$ gleich oder verschieden sind und Wasserstoff, ($C_1$ bis $C_6$)-Alkyl, Trifluormethyl, Halogen, Methoxycarbonyl, Ethoxycarbonyl, ($C_1$ bis $C_5$)-Alkoxy oder ($C_1$ bis $C_4$)-Alkanoyl bedeuten,

$R^3$, $R^4$ und $R^5$ gleich oder verschieden sind und Wasserstoff, Methyl, Methoxy, Ethoxy, Methoxyethoxy oder Ethoxyethoxy bedeuten und

R Wasserstoff oder eine Gruppe $SR^6$ bedeuten, worin $R^6$ für das freie, ungepaarte Elektron des 12-Sulfenylradikals, Wasserstoff, ($C_1$ bis $C_6$)-Alkyl, das gegebenenfalls mit ($C_1$ bis $C_4$)-Alkoxy oder ein- oder mehrfach mit ($C_6$ bis $C_{10}$)-Aryl substituierte sein kann, ($C_2$ bis $C_6$)-Alkenyl, ($C_2$ bis $C_6$)-Alkinyl, ($C_1$ bis $C_7$)-Alkanoyl, ($C_5$ bis $C_7$)-Cycloalkanoyl, Benzoyl, das gegebenenfalls ein- oder mehrfach vorzugsweise ein- bis dreifach mit ($C_1$ bis $C_7$)-Alkyl, ($C_1$ bis $C_5$)-Alkoxy, ($C_1$ bis $C_4$)Alkoxycarbonyl, Cyano, Trifluormethyl, und/oder Halogen substituiert ist, Furoyl, Phenacyl, Phenoxyacetyl, oder Phenylacetyl, wobei die drei letzteren wie Benzoyl substituiert sein können, oder eine andere gebräuchliche Thiol-Schutzgruppe steht, sowie deren physiologisch verträglichen Salze.

Die Erfindung betrifft außerdem Isomerengemische von Verbindungen der Formel I, die beispielsweise dadurch entstehen, daß man im Benzimidazolteil unsymmetrisch substituierte Verbindungen mit $R^1 \neq R^2$ der Formel III (s. S. 17) in Gegenwart von Säuren umlagert.

Schutzgruppen, wie sie normalerweise zur Protektion von Thiolen Verwendung finden, sind beispielsweise in: Theodora W. Greene, Protective Groups in Organic Synthesis, Wiley-Interscience, New York, 1981, Seite 193 ff. beschrieben. Bevorzugt sind solche Thiol-Schutzgruppen, die in Form physiologisch unbedenklicher Verbindungen abgespalten werden können.

Unter physiologisch verträglichen Salzen der Verbindungen der Formel I werden beispielsweise wasserlösliche und wasserunlösliche Säureadditionssalze, wie das Hydrochlorid, Hydrobromid, Hydrojodid, Phosphat, Nitrat, Sulfat, Acetat, Citrat, Gluconat, Benzoat, Butyrat, Sulfosalicylat, Maleat, Laurat, Malat, Fumarat, Succinat, Oxalat, Tartrat, Stearat, Methansulfonat und Toluolsulfonat verstanden.

Von Verbindungen der Formel I, in denen $R^6$ Wasserstoff bedeutet, lassen sich andererseits durch Umsetzung mit geeigneten Deprotonierungsmitteln (z. B. anorganischen oder organischen Basen, wie Alkali oder Erdalkalihydroxiden oder -amiden, primären, sekundären oder tertiären aliphatischen Aminen, Guanidin oder seinen Derivaten) Basenadditionssalze herstellen.

Alkylreste $R^1$ und $R^2$ sind geeignete Alkylgruppen mit bis zu 6 C-Atomen, vorzugsweise bis zu 4 C-Atomen, also Methyl, Ethyl, n-Propyl, iso-Propyl, n-Butyl, iso-Butyl, tert.-Butyl.

Halogensubstituenten sind Fluor, Chlor, Brom und Jod.

Alkoxyreste $R^1$ und $R^2$ sind geeignete Alkoxygruppen mit bis zu 5 C-Atomen, vorzugsweise bis zu 3 C-Atomen, also Methoxy, Ethoxy, n-Propoxy oder iso-Propoxy.

Alkanoylreste $R^1$ und $R^2$ haben vorzugsweise bis zu 4 C-Atome und sind z. B. Formyl, Acetyl, Propionyl oder Butyryl, vorzugsweise Acetyl.

Der Alkylrest $R^6$ ist eine geeignete Alkylgruppe mit bis zu 6 C-Atomen, vorzugsweise bis zu 4 C-Atomen, also Methyl, Ethyl, n-Propyl, iso-Propyl, n-Butyl, iso-Butyl, tert.-Butyl. Als substituierte Alkylgruppen kommen bevorzugt Methoxymethyl und Ethoxymethyl bzw. Benzyl-, Benzhydryl- und Triphenylmethylgruppen in Frage. Alkenyl- und Alkinylreste $R^6$ enthalten vorzugsweise bis zu 3 C-Atomen, beispielsweise seien Allyl- und Propargylgruppen genannt. Alkanoylreste $R^6$ haben vorzugsweise bis zu 7 C-Atome und sind beispielsweise Formyl, Acetyl, Propionyl, Butyryl und Pivaloyl. Als substituierte Benzoylreste $R^6$ kommen insbesondere ein- oder mehrfach mit ($C_1$ bis $C_4$)-Alkyl-, ($C_1$ bis $C_4$)-Alkoxy-, ($C_1$ bis $C_4$)-Alkoxycarbonyl- oder Halogenresten aber auch mit Cyano oder Trifluormethyl substituierte Reste in Frage. Cycloalkanoyl ist vorzugsweise

Cyclohexylcarbonyl.

Als erfindungsgemäße Verbindungen seien genannt, ohne die Erfindung auf diese zu beschränken:

Pyrido(1,2-c)imidazo(1,2-a)benzimidazol-12-sulfenyl,
12-Mercapto-pyrido(1,2-c)imidazo(1,2-a)benzimidazol,
12-Methylthio-pyrido(1,2-c)imidazo(1,2-a)benzimidazol,
12-Ethylthio-pyrido(1,2-c)imidazo(1,2-a)benzimidazol,
12-n-Propylthio-pyrido(1,2-c)imidazo(1,2-a)benzimidazol,
12-tert.-Butylthio-pyrido(1,2-c)imidazo(1,2-a)benzimidazol,
12-Methoxymethylthio-pyrido(1,2-c)imidazo(1,2-a)benzimidazol,
12-Ethoxymethylthio-pyrido(1,2-c)imidazo(1,2-a)benzimidazol,
12-Benzylthio-pyrido(1,2-c)imidazo(1,2-a)benzimidazol,
12-Benzhydrylthio-pyrido(1,2-c)imidazo(1,2-a)benzimidazol,
12-Tritylthio-pyrido(1,2-c)imidazo(1,2-a)benzimidazol,
12-Formylthio-pyrido(1,2-c)imidazo(1,2-a)benzimidazol,
Acetylthio-pyrido(1,2-c)imidazo(1,2-a)benzimidazol,
12-Propionylthio-pyrido(1,2-c)imidazo(1,2-a)benzimidazol,
12-Butyrylthio-pyrido(1,2-c)imidazo(1,2-a)benzimidazol,
12-Pivaloylthio-pyrido(1,2-c)imidazo(1,2-a)benzimidazol,
12-Benzoylthio-pyrido(1,2-c)imidazo(1,2-a)benzimidazol,
-pyrido(1,2-c)imidazo(1,2-a)benzimidazol,
12-(3-Methoxybenzoyl)thio-pyrido(1,2-c)imidazo(1,2-a)benzimidazol,
12-(3-Chlorbenzoyl)thio-pyrido(1,2-c)imidazo(1,2-a)benzimidazol,
12-Phenylacetylthio-pyrido(1,2-c)imidazo(1,2-a)benzimidazol,
8-Methyl-pyrido(1,2-c)imidazo(1,2-a)benzimidazol-12-sulfenyl,
9-Methyl-pyrido(1,2-c)imidazo(1,2-a)benzimidazol-12-sulfenyl,
8-tert.-Butyl-pyrido(1,2-c)imidazo(1,2-a)benzimidazol-12-sulfenyl,
9-tert.-Butyl-pyrido(1,2-c)imidazo(1,2-a)benzimidazol-12-sulfenyl,
3-Ethyl-pyrido(1,2-c)imidazo(1,2-a)benzimidazol-12-sulfenyl,
2-Methyl-pyrido(1,2-c)imidazo(1,2-a)benzimidazol-12-sulfenyl,
2-Methoxy-pyrido(1,2-c)imidazo(1,2-a)benzimidazol-12-sulfenyl,
8-Ethoxycarbonyl-pyrido(1,2-c)imidazo(1,2-a)benzimidazol-12-sulfenyl,
9-Ethoxycarbonyl-2-methoxy-pyrido(1,2-c)imidazo(1,2-a)benzimidazol-12-sulfenyl,
9-Ethoxycarbonyl-2-methoxy-pyrido(1,2-c)imidazo(1,2-a)benzimidazol-12-sulfenyl,
12-Acetylthio-8-methyl-pyrido(1,2-c)imidazo(1,2-a)benzimidazol,
12-Acetylthio-9-methyl-pyrido(1,2-c)imidazo(1,2-a)benzimidazol,
12-Acetylthio-8-tert.-butyl-pyrido(1,2-c)imidazo(1,2-a)benzimidazol,
12-Acetylthio-9-tert.-butyl-pyrido(1,2-c)imidazo(1,2-a)benzimidazol,
12-Acetylthio-3-ethyl-pyrido(1,2-c)imidazo(1,2-a)benzimidazol,
12-Acetylthio-2-methyl-pyrido(1,2-c)imidazo(1,2-a)benzimidazol,
12-Acetylthio-2-methoxy-pyrido(1,2-c)imidazo(1,2-a)benzimidazol,
12-Acetylthio-8-ethoxycarbonyl-pyrido(1,2-c)imidazo(1,2-a)benzimidazol,
12-Acetylthio-9-ethoxycarbonyl-pyrido(1,2-c)imidazo(1,2-a)benzimidazol,
12-Acetylthio-8-ethoxycarbonyl-2-methoxy-pyrido(1,2-c)imidazo(1,2-a)benzimidazol,
12-Acetylthio-9-ethoxycarbonyl-2-methoxy-pyrido(1,2-c)imidazo(1,2-a)benzimidazol,

2,8-Dimethoxy-1,3-dimethyl-pyrido(1,2-c)imidazo(1,2-a)benzimidazol-12-sulfenyl
2,8-Dimethoxy-1,3-dimethyl-12-mercapto-pyrido(1,2-c)imidazo(1,2-a)benzimidazol,
2,8-Dimethoxy-1,3-dimethyl-12-methylthio-pyrido(1,2-c)imidazo(1,2-a)benzimidazol,
2,8-Dimethoxy-1,3-dimethyl-12-methylthio-pyrido(1,2-c)imidazo(1,2-a)benzimidazol,
2,8-Dimethoxy-1,3-dimethyl-12-ethylthio-pyrido(1,2-c)imidazo(1,2-a)benzimidazol,
2,8-Dimethoxy-1,3-dimethyl-12-n-propylthio-pyrido(1,2-c)imidazo(1,2-a)benzimidazol,
2,8-Dimethoxy-1,3-dimethyl-12-tert.-butylthio-pyrido(1,2-c)imidazo(1,2-a)benzimidazol,
2,8-Dimethoxy-1,3-dimethyl-12-methoxymethylthio-pyrido(1,2-c)imidazo(1,2-a)benzimidazol,
2,8-Dimethoxy-1,3-dimethyl-12-ethoxymethylthio-pyrido(1,2-c)imidazo(1,2-a)benzimidazol,
2,8-Dimethoxy-1,3-dimethyl-12-benzylthio-pyrido(1,2-c)imidazo(1,2-a)benzimidazol,
2,8-Dimethoxy-1,3-dimethyl-12-benzhydrylthio-pyrido(1,2-c)imidazo(1,2-a)benzimidazol,
2,8-Dimethoxy-1,3-dimethyl-12-tritylthio-pyrido(1,2-c)imidazo(1,2-a)benzimidazol,
2,8-Dimethoxy-1,3-dimethyl-12-formylthio-pyrido(1,2-c)imidazo(1,2-a)benzimidazol,
2,8-Dimethoxy-1,3-dimethyl-12-acetylthio-pyrido(1,2-c)imidazo(1,2-a)benzimidazol,
2,8-Dimethoxy-1,3-dimethyl-12-propionylthio-pyrido(1,2-c)imidazo(1,2-a)benzimidazol,
2,8-Dimethoxy-1,3-dimethyl-12-butyrylthio-pyrido(1,2-c)imidazo(1,2-a)benzimidazol,
2,8-Dimethoxy-1,3-dimethyl-12-pivaloylthio-pyrido(1,2-c)imidazo(1,2-a)benzimidazol,
2,8-Dimethoxy-1,3-dimethyl-12-benzoylthio-pyrido(1,2-c)imidazo(1,2-a)benzimidazol,
2,8-Dimethoxy-1,3-dimethyl-12-(3-methoxybenzoyl)thio-pyrido(1,2-c)imidazo(1,2-a)benzimidazol,

2,8-Dimethoxy-1,3-dimethyl-12-(3-chlorbenzoyl)thio-pyrido(1,2-c)imidazo(1,2-a)benzimidazol,
2,8-Dimethoxy-1,3-dimethyl-12-phenylacetylthio-pyrido(1,2-c)imidazo(1,2-a)benzimidazol,
2,9-Dimethoxy-1,3-dimethyl-pyrido(1,2-c)imidazo(1,2-a)benzimidazol-12-sulfenyl,
12-mercapto-pyrido(1,2-c)imidazo(1,2-a)benzimidazol,
2,9-Dimethoxy-1,3-dimethyl-12-methylthio-pyrido(1,2-c)imidazo(1,2-a)benzimidazol,
2,9-Dimethoxy-1,3-dimethyl-12-ethylthio-pyrido(1,2-c)imidazo(1,2-a)benzimidazol,
2,9-Dimethoxy-1,3-dimethyl-12-n-propylthio-pyrido(1,2-c)imidazo(1,2-a)benzimidazol,
2,9-Dimethoxy-1,3-dimethyl-12-tert.-butylthio-pyrido(1,2-c)imidazo(1,2-a)benzimidazol,
2,9-Dimethoxy-1,3-dimethyl-12-methoxymethylthio-pyrido(1,2-c)imidazo(1,2-a)benzimidazol,
2,9-Dimethoxy-1,3-dimethyl-12-ethoxymethylthio-pyrido(1,2-c)imidazo(1,2-a)benzimidazol,
2,9-Dimethoxy-1,3-dimethyl-12-benzylthio-pyrido(1,2-c)imidazo(1,2-a)benzimidazol,
2,9-Dimethoxy-1,3-dimethyl-12-benzhydrylthio-pyrido(1,2-c)imidazo(1,2-a)benzimidazol,
2,9-Dimethoxy-1,3-dimethyl-12-tritylthio-pyrido(1,2-c)imidazo(1,2-a)benzimidazol,
2,9-Dimethoxy-1,3-dimethyl-12-formylthio-pyrido(1,2-c)imidazo(1,2-a)benzimidazol,
2,9-Dimethoxy-1,3-dimethyl-12-acetylthio-pyrido(1,2-c)imidazo(1,2-a)benzimidazol,
2,9-Dimethoxy-1,3-dimethyl-12-propionylthio-pyrido(1,2-c)imidazo(1,2-a)benzimidazol,
2,9-Dimethoxy-1,3-dimethyl-12-butyrylthio-pyrido(1,2-c)imidazo(1,2-a)benzimidazol,
2,9-Dimethoxy-1,3-dimethyl-12-pivaloylthio-pyrido(1,2-c)imidazo(1,2-a)benzimidazol,
2,9-Dimethoxy-1,3-dimethyl-12-benzoylthio-pyrido(1,2-c)imidazo(1,2-a)benzimidazol,
2,9-Dimethoxy-1,3-dimethyl-12-(3-methoxybenzoyl)thio-pyrido(1,2-c)imidazo(1,2-a)benzimidazol,
2,9-Dimethoxy-1,3-dimethyl-12-(3-chlorbenzoyl)thio-pyrido(1,2-c)imidazo(1,2-a)benzimidazol,
2,9-Dimethoxy-1,3-dimethyl-12-(3-chlorbenzoyl)thio-pyrido(1,2-c)imidazo(1,2-a)benzimidazol,
2,9-Dimethoxy-1,3-dimethyl-12-phenylacetylthio-pyrido(1,2-c)imidazo(1,2-a)benzimidazol,
2,9-Dimethoxy-1,3-dimethyl-pyrido(1,2-c)imidazo(1,2-a)benzimidazol,
2-Methoxy-1,3-dimethyl-8-trifluormethyl-pyrido(1,2-c)imidazo(1,2-a)benzimidazol-12-sulfenyl,
2-Methoxy-1,3-dimethyl-8-trifluormethyl-12-mercapto-pyrido(1,2-c)imidazo(1,2-a)benzimidazol,
2-Methoxy-1,3-dimethyl-8-trifluormethyl-12-methylthio-pyrido(1,2-c)imidazo(1,2-a)benzimidazol,
2-Methoxy-1,3-dimethyl-8-trifluormethyl-12-ethylthio-pyrido(1,2-c)imidazo(1,2-a)benzimidazol,
2-Methoxy-1,3-dimethyl-8-trifluormethyl-12-n-propylthio-pyrido(1,2-c)imidazo(1,2-a)benzimidazol,
2-Methoxy-1,3-dimethyl-8-trifluormethyl-12-tert.-butylthio-pyrido(1,2-c)imidazo(1,2-a)benzimidazol,
2-Methoxy-1,3-dimethyl-8-trifluormethyl-12-methoxymethylthio-pyrido(1,2-c)imidazo(1,2-a)benzimidazol,
2-Methoxy-1,3-dimethyl-8-trifluormethyl-12-ethoxymethylthio-pyrido(1,2-c)imidazo(1,2-a)benzimidazol,
2-Methoxy-1,3-dimethyl-8-trifluormethyl-12-benzylthio-pyrido(1,2-c)imidazo(1,2-a)benzimidazol,
2-Methoxy-1,3-dimethyl-8-trifluormethyl-12-tert.-butylthio-pyrido(1,2-c)imidazo(1,2-a)benzimidazol,
2-Methoxy-1,3-dimethyl-8-trifluormethyl-12-methoxymethylthio-pyrido(1,2-c)imidazo(1,2-a)benzimidazol,
2-Methoxy-1,3-dimethyl-8-trifluormethyl-12-ethoxymethylthio-pyrido(1,2-c)imidazo(1,2-a)benzimidazol,
2-Methoxy-1,3-dimethyl-8-trifluormethyl-12-benzylthio-pyrido(1,2-c)imidazo(1,2-a)benzimidazol,
2-Methoxy-1,3-dimethyl-8-trifluormethyl-12-benzhydrylthio-pyrido(1,2-c)imidazo(1,2-a)benzimidazol,
2-Methoxy-1,3-dimethyl-8-trifluormethyl-12-tritylthio-pyrido(1,2-c)imidazo(1,2-a)benzimidazol,
2-Methoxy-1,3-dimethyl-8-trifluormethyl-12-formylthio-pyrido(1,2-c)imidazo(1,2-a)benzimidazol,
2-Methoxy-1,3-dimethyl-8-trifluormethyl-12-acetylthio-pyrido(1,2-c)imidazo(1,2-a)benzimidazol,
2-Methoxy-1,3-dimethyl-8-trifluormethyl-12-propionylthio-pyrido(1,2-c)imidazo(1,2-a)benzimidazol,
2-Methoxy-1,3-dimethyl-8-trifluormethyl-12-butyrylthio-pyrido(1,2-c)imidazo(1,2-a)benzimidazol,
2-Methoxy-1,3-dimethyl-8-trifluormethyl-12-pivaloylthio-pyrido(1,2-c)imidazo(1,2-a)benzimidazol,
2-Methoxy-1,3-dimethyl-8-trifluormethyl-12-benzoylthio-pyrido(1,2-c)imidazo(1,2-a)benzimidazol,
2-Methoxy-1,3-dimethyl-8-trifluormethyl-12-(3-methoxybenzoyl)thio-pyrido(1,2-c)imidazo(1,2-a)benzimidazol,
2-Methoxy-1,3-dimethyl-8-trifluormethyl-12-(3-chlorbenzoyl)thio-pyrido(1,2-c)imidazo(1,2-a)benzimidazol,
2-Methoxy-1,3-dimethyl-8-trifluormethyl-12-phenylacetylthio-pyrido(1,2-c)imidazo(1,2-a)benzimidazol,

2-Methoxy-1,3-dimethyl-9-trifluormethyl-pyrido(1,2-c)imidazo(1,2-a)benzimidazol-12-sulfenyl,
2-Methoxy-1,3-dimethyl-8-trifluormethyl-12-mercapto-pyrido(1,2-c)imidazo(1,2-a)benzimidazol,
2-Methoxy-1,3-dimethyl-8-trifluormethyl-12-Methylthio-pyrido(1,2-c)imidazo(1,2-a)benzimidazol,
2-Methoxy-1,3-dimethyl-9-trifluormethyl-12-ethylthio-pyrido(1,2-c)imidazo(1,2-a)benzimidazol,
2-Methoxy-1,3-dimethyl-9-trifluormethyl-12-n-propylthio-pyrido(1,2-c)imidazo(1,2-a)benzimidazol,
2-Methoxy-1,3-dimethyl-9-trifluormethyl-12-tert.-butylthio-pyrido(1,2-c)imidazo(1,2-a)benzimidazol,
2-Methoxy-1,3-dimethyl-9-trifluormethyl-12-methoxymethylthio-pyrido(1,2-c)imidazo(1,2-a)benzimidazol,
2-Methoxy-1,3-dimethyl-9-trifluormethyl-12-ethoxymethylthio-pyrido(1,2-c)imidazo(1,2-a)benzimidazol,
2-Methoxy-1,3-dimethyl-9-trifluormethyl-12-benzylthio-pyrido(1,2-c)imidazo(1,2-a)benzimidazol,
2-Methoxy-1,3-dimethyl-9-trifluormethyl-12-benzhydrylthio-pyrido(1,2-c)imidazo(1,2-a)benzimidazol,
2-Methoxy-1,3-dimethyi-9-trifluormethyl-12-tritylthio-pyrido(1,2-c)imidazo(1,2-a)benzimidazol,
2-Methoxy-1,3-dimethyl-9-trifluormethyl-12-formylthio-pyrido(1,2-c)imidazo(1,2-a)benzimidazol,
2-Methoxy-1,3-dimethyl-9-trifluormethyl-12-acetylthio-pyrido(1,2-c)imidazo(1,2-a)benzimidazol,
2-Methoxy-1,3-dimethyl-9-trifluormethyl-12-propionylthio-pyrido(1,2-c)imidazo(1,2-a)benzimidazol,
2-Methoxy-1,3-dimethyl-9-trifluormethyl-12-butyrylthio-pyrido(1,2-c)imidazo(1,2-a)benzimidazol,
2-Methoxy-1,3-dimethyl-9-trifluormethyl-12-pivaloylthio-pyrido(1,2-c)imidazo(1,2-a)benzimidazol,

2-Methoxy-1,3-dimethyl-9-trifluormethyl-12-benzoylthio-pyrido(1,2-c)imidazo(1,2-a)benzimidazol,
2-Methoxy-1,3-dimethyl-9-trifluormethyl-12-(3-methoxybenzoyl)thio-pyrido(1,2-c)imidazo(1,2-a)benzimidazol,
2-Methoxy-1,3-dimethyl-9-trifluormethyl-12-(3-chlorbenzoyl)thio-pyrido(1,2-c)imidazo(1,2-a)benzimidazol,
2-Methoxy-1,3-dimethyl-9-trifluormethyl-12-phenylacetylthio-pyrido(1,2-c)imidazo(1,2-a)benzimidazol,
2-Methoxy-1,3-dimethyl-9-trifluormethyl-pyrido(1,2-c)imidazo(1,2-a)benzimidazol,
8-Methoxycarbonyl-1,9-dimethyl-pyrido(1,2-c)imidazo(1,2-a)benzimidazol-12-sulfenyl,
8-Methoxycarbonyl-1,9-dimethyl-12-Mercapto-pyrido(1,2-c)imidazo(1,2-a)benzimidazol,
8-Methoxycarbonyl-1,9-dimethyl-12-Methylthio-pyrido(1,2-c)imidazo(1,2-a)benzimidazol,
8-Methoxycarbonyl-1,9-dimethyl-12-Ethylthio-pyrido(1,2-c)imidazo(1,2-a)benzimidazol,
8-Methoxycarbonyl-1,9-dimethyl-12-n-Propylthio-pyrido(1,2-c)imidazo(1,2-a)benzimidazol,
8-Methoxycarbonyl-1,9-dimethyl-12-tert.-butylthio-pyrido(1,2-c)imidazo(1,2-a)benzimidazol,
8-Methoxycarbonyl-1,9-dimethyl-12-methoxymethylthio-pyrido(1,2-c)imidazo(1,2-a)benzimidazol,
8-Methoxycarbonyl-1,9-dimethyl-12-ethoxymethylthio-pyrido(1,2-c)imidazo(1,2-a)benzimidazol,
8-Methoxycarbonyl-1,9-dimethyl-12-benzylthio-pyrido(1,2-c)imidazo(1,2-a)benzimidazol,
8-Methoxycarbonyl-1,9-dimethyl-12-benzhydrylthio-pyrido(1,2-c)imidazo(1,2-a)benzimidazol,
8-Methoxycarbonyl-1,9-dimethyl-12-tritylthio-pyrido(1,2-c)imidazo(1,2-a)benzimidazol,
8-Methoxycarbonyl-1,9-dimethyl-12-formylthio-pyrido(1,2-c)imidazo(1,2-a)benzimidazol,
8-Methoxycarbonyl-1,9-dimethyl-12-acetylthio-pyrido(1,2-c)imidazo(1,2-a)benzimidazol,
8-Methoxycarbonyl-1,9-dimethyl-12-propionylthio-pyrido(1,2-c)imidazo(1,2-a)benzimidazol,
8-Methoxycarbonyl-1,9-dimethyl-12-butyrylthio-pyrido(1,2-c)imidazo(1,2-a)benzimidazol,
8-Methoxycarbonyl-1,9-dimethyl-12-pivaloylthio-pyrido(1,2-c)imidazo(1,2-a)benzimidazol,
8-Methoxycarbonyl-1,9-dimethyl-12-benzoylthio-pyrido(1,2-c)imidazo(1,2-a)benzimidazol,
8-Methoxycarbonyl-1,9-dimethyl-12-(3-methoxybenzoyl)thio-pyrido(1,2-c)imidazo(1,2-a)benzimidazol,
8-Methoxycarbonyl-1,9-dimethyl-12-(3-chlorbenzoyl)thio-pyrido(1,2-c)imidazo(1,2-a)benzimidazol,
8-Methoxycarbonyl-1,9-dimethyl-12-phenylacetylthio-pyrido(1,2-c)imidazo(1,2-a)benzimidazol,
8-Methoxycarbonyl-1,9-dimethyl-pyrido(1,2-c)imidazo(1,2-a)benzimidazol,
9-Methoxycarbonyl-1,8-dimethyl-pyrido(1,2-c)imidazo(1,2-a)benzimidazol-12-sulfenyl,
9-Methoxycarbonyl-1,8-dimethyl-12-mercapto-pyrido(1,2-c)imidazo(1,2-a)benzimidazol,
9-Methoxycarbonyl-1,8-dimethyl-12-methylthio-pyrido(1,2-c)imidazo(1,2-a)benzimidazol,
9-Methoxycarbonyl-1,8-dimethyl-12-ethylthio-pyrido(1,2-c)imidazo(1,2-a)benzimidazol,
9-Methoxycarbonyl-1,8-dimethyl-12-n-propylthio-pyrido(1,2-c)imidazo(1,2-a)benzimidazol,
9-Methoxycarbonyl-1,8-dimethyl-12-tert.-butylthio-pyrido(1,2-c)imidazo(1,2-a)benzimidazol,
9-Methoxycarbonyl-1,8-dimethyl-12-methoxymethylthio-pyrido(1,2-c)imidazo(1,2-a)benzimidazol,
9-Methoxycarbonyl-1,8-dimethyl-12-ethoxymethylthio-pyrido(1,2-c)imidazo(1,2-a)benzimidazol,
9-Methoxycarbonyl-1,8-dimethyl-12-benzylthio-pyrido(1,2-c)imidazo(1,2-a)benzimidazol,
9-Methoxycarbonyl-1,8-dimethyl-12-benzhydrylthio-pyrido(1,2-c)imidazo(1,2-a)benzimidazol,
9-Methoxycarbonyl-1,8-dimethyl-12-tritylthio-pyrido(1,2-c)imidazo(1,2-a)benzimidazol,
9-Methoxycarbonyl-1,8-dimethyl-12-formylthio-pyrido(1,2-c)imidazo(1,2-a)benzimidazol,
9-Methoxycarbonyl-1,8-dimethyl-12-acetylthio-pyrido(1,2-c)imidazo(1,2-a)benzimidazol,
9-Methoxycarbonyl-1,8-dimethyl-12-propionylthio-pyrido(1,2-c)imidazo(1,2-a)benzimidazol,
9-Methoxycarbonyl-1,8-dimethyl-12-butyrylthio-pyrido(1,2-c)imidazo(1,2-a)benzimidazol,
9-Methoxycarbonyl-1,8-dimethyl-12-pivaloylthio-pyrido(1,2-c)imidazo(1,2-a)benzimidazol,
9-Methoxycarbonyl-1,8-dimethyl-12-(3-chlorbenzoyl)thio-pyrido(1,2-c)imidazo(1,2-a)benzimidazol,
9-Methoxycarbonyl-1,8-dimethyl-12-phenylacetylthio-pyrido(1,2-c)imidazo(1,2-a)benzimidazol.

Die Erfindung betrifft weiterhin ein Verfahren zur Herstellung von Verbindungen der Formel I, das dadurch gekennzeichnet ist, daß man

$a_1$) zur Herstellung von Verbindungen der Formel I mit R = Wasserstoff Verbindungen der Formel II,

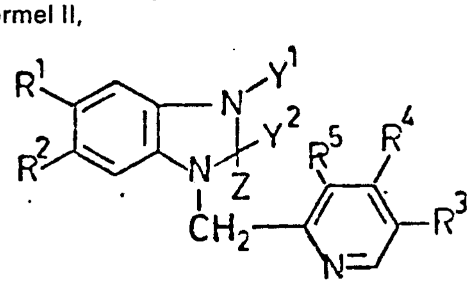

(II)

in welcher $R^1$ bis $R^5$ wie oben definiert sind und entweder $Y^1 + Y^2$ zusammen für eine Bindung und Z für Abgangsgruppen, wie Halogen, vorzugsweise Chlor stehen oder $Y^1$ für Wasserstoff und $Y^2 + Z$ zusammen für eine Oxogruppe stehen, cyclisiert,

$a_2$) Verbindungen der Formel III a

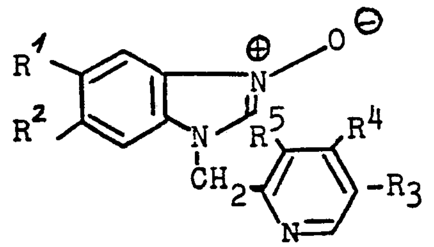

(III a)

in welcher $R^1$ bis $R^5$ wie oben definiert sind, vorzugsweise unter Verwendung von Trifluoressigsäureanhydrid, Toluolsulfonsäurechlorid, Methansulfonsäurechlorid u.ä. cyclisiert,

b) zur Herstellung von Verbindungen der Formel I mit R = Sulfenyl

$b_1$) 2-Benzimidazol-2'-pyridyl-sulfoxide der Formel III,

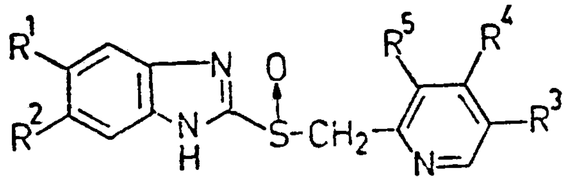

(III)

in welcher $R^1$ bis $R^5$ wie oben definiert sind, mit einer Säure behandelt, wobei im Falle $R^1 \neq R^2$ Gemische stellungsisomerer Verbindungen entstehen,

b) Verbindungen der Formel I in welcher $R^1$ bis $R^5$ wie oben definiert sind und R Wasserstoff bedeutet, mit elementaren Schwefel oder mit $S_2Cl_2$ in einem Lösungsmittel umsetzt oder

c) zur Herstellung von Verbindungen der Formel I mit R = Wasserstoff oder Sulfenyl

Verbindungen der Formel I, in welcher $R^1$ bis $R^5$ wie oben definiert sind und R Sulfenyl bedeutet, reduziert zu Verbindungen der Formel I, worin $R^6$ Wasserstoff ist und diese gewünschtenfalls umsetzt mit Verbindungen der Formel $R^6X$, worin $R^6$ die oben definierten Bedeutungen außer einem radikalischen Elektron und Wasserstoff hat und X eine Abgangsgruppe ist, und die nach $a_1$) - c) erhaltenen Verbindungen gegebenenfalls in ihre physiologisch verträglichen Salze überführt.

Bei der Verfahrensvariante $a_1$) wird vorzugsweise ein Benzimidazolderivat der Formel VII

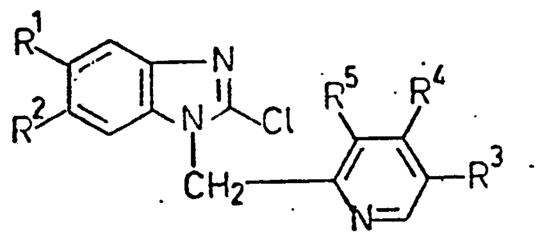

(VII)

in der $R^1$ bis $R^5$ wie oben definiert sind, unter geeigneten Bedingungen, wie z. B. Erwärmen in einem organischen Lösungsmittel in Gegenwart einer starken Säure (beispielsweise in ethanolischer Salzsäure) auf eine Temperatur unterhalb des Siedepunktes cyclisiert; oder es wird ein Benzimidazolonderivat der Formel VIII

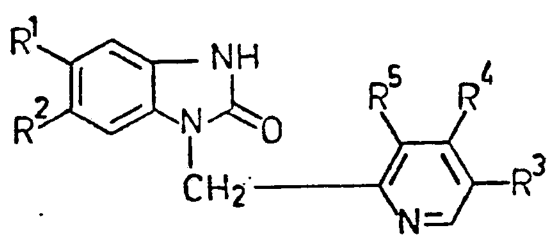

(VIII)

in der $R^1$ bis $R^5$ wie in oben definiert sind, unter geeigneten Bedingungen, wie z. B. Säure oder deren Halogenid (wie Polyphosphorsäure oder Phosphoroxychlorid), vorzugsweise durch Kochen mit Phosphoroxychlorid am Rückfluß cyclisiert;

oder es wird ein Benzimidazol-N-oxid-derivat der Formel III a

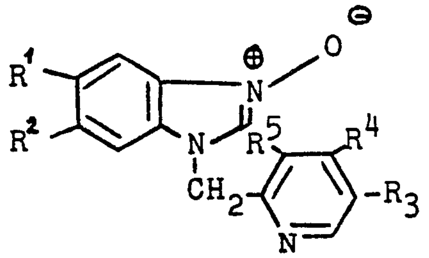

(III a)

in der $R^1$-$R^5$ wie oben definiert sind, unter geeigneten Bedingungen cyclisiert, z. B. in inerten Lösungsmitteln wie $CH_2Cl_2$, $CHCl_3$, $CCl_4$, Ether, THF durch Verwendung von Trifluoressigsäureanhydrid, TsCl, MesCl zwischen 0 - 100° C, vorzugsweise bei Raumtemperatur.

Bei der Verfahrensvariante $b_1$) führt man die Umlagerung vorzugsweise in Lösungsmitteln, insbesondere in wäßriger Lösung in Gegenwart von anorganischen oder organischen Säuren wie beispielsweise HCl, $H_2SO_4$ $HC10_4$ $CF_3COOH$ oder ähnlich geeigneten Säuren oder deren Gemischen vorzugsweise bei pH-Werten um pH 1 bei Temperaturen zwischen 0 und 60° C durch.

Die Ausgangsverbindungen der Formel III und Verfahren zu ihrer Herstellung sind unter anderem aus den eingangs genannten Offenlegungsschriften DE-A-2 548 340, EP-A-5129 und DE-A-3 240 248 bekannt.

Die Umsetzung von Verbindungen der Formel I, R = Wasserstoff gemäß Verfahrensvariante $b_2$) mit elementarem Schwefel wird vorzugsweise ohne Lösungsmittel bei Temperaturen oberhalb des Schmelzpunktes von Schwefel durchgeführt, während man die Umsetzung mit $S_2Cl_2$ in einem inerten organischen Lösungsmittel wie Diethylether, THF, Methylenchlorid oder Chloroform oder deren Gemischen vorzugsweise zwischen 0° C und Raumtemperatur durchführt.

Das Verfahren gemäß Variante c) zeichnet sich dadurch aus, daß die Reduktion der Verbindung der Formel I, R = Sulfenyl in organischen Lösungsmitteln, vorzugsweise in Ethern wie Diethylether oder THF, denen man zweckmäßigerweise etwas Wasser oder Alkohol zusetzt, mit komplexen Hydriden oder anderen geeigneten Reduktionsmitteln durchgeführt wird, bei Temperaturen zwischen 0 und 60° C, vorzugsweise bei Raumtemperatur. Wegen der leichten Oxidierbarkeit der entstehenden Thiolverbindung arbeitet man zweckmäßigerweise unter Schutzgasatmosphäre. Das Alkylierungs- oder Acylierungsmittel $R_6X$ wird gegebenenfalls direkt nach beendeter Reduktion zugegeben, wobei gegebenenfalls die Reaktionslösung gekühlt werden muß. Welche Abgangsgruppen X geeignet sind, ist dem Fachmann aufgrund seines Fachwissens geläufig. In Frage kommen bei Alkylierungsmitteln beispielsweise Halogene oder Tosylate, bei Acylierungsmitteln ebenfalls Halogene, Carboxylate oder Toluolsulfonate.

Das Produkt läßt sich durch Säulenchromatographie oder Kristallisation isolieren und reinigen.

Die Erfindung betrifft auch Verbindungen der Formel II, in der $R^1$ bis $R^5$, $Y^1$, $Y^2$ und Z wie oben definiert sind und ein Verfahren zu deren Herstellung, das dadurch gekennzeichnet ist, daß man

a) zur Herstellung einer Verbindung der Formel II mit $Y^1$ und $Y^2$ = Bindung und Z = Abgangsgruppe, vorzugsweise Halogen, wie Chlor eine Verbindung der Formel IV

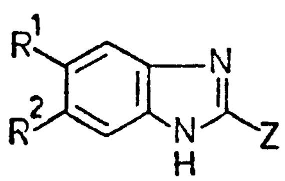

(IV)

in welcher $R^1$ $R^2$ und Z wie oben definiert sind, umsetzt mit einem gegebenenfalls substituierten Picolylhalogenid der Formel V

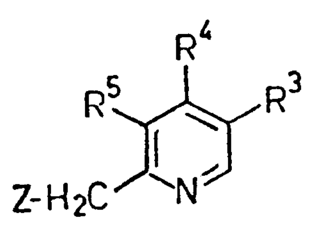

(V)

in welcher $R^3$ bis $R^5$ und Z wie oben definiert sind oder

b) zur Herstellung einer Verbindung der Formel II mit $Y^1$ = Wasserstoff und $Y^2$ + Z = Oxo eine Verbindung der Formel VI

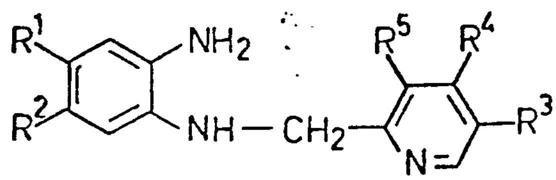

(VI)

in welcher $R^1$ bis $R^5$ wie oben definiert sind, mit einem Kohlensäurederivat wie Harnstoff umsetzt.

Die Ausgangsverbindungen der Formeln IV und V sind literaturbekannt oder werden nach bekannten Analogieverfahren hergestellt.

Substituierte o-Phenylendiamine der Formel VI werden beispielsweise hergestellt durch Umsetzung eines geeignet substituierten o-Nitroanilins mit einem geeignet substituierten 2-Pyridinaldehyd zur Schiffschen Base der Formel IX

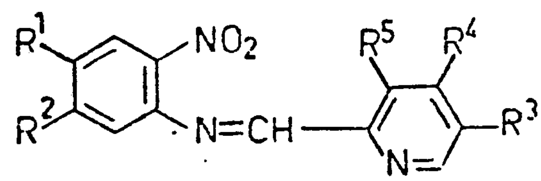

(IX)

wobei $R^1$ bis $R^5$ die obengenannten Bedeutungen haben, anschließende Reduktion zum substituierten o-Nitroamin der Formel X,

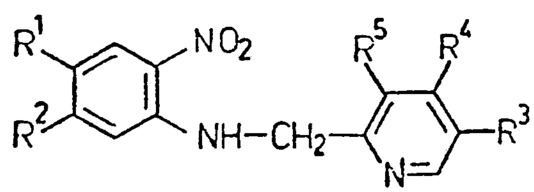

(X)

und Hydrierung zum substituierten o-Phenylendiamin der Formel VI.

Die Reduktion der Verbindungen der Formel IX erfolgt zweckmäßig mit $NaBH_4$ oder einem anderen, geeigneten Hydrid vorzugsweise in Ethanol unter Rückfluß. Die Nitrogruppe der Verbindungen der Formel X wird mit geeigneten Reduktionsmitteln, wie $H_2$/Raney-Nickel, $SnCl_2$/HCl oder Zn/HCl (vgl. hierzu Houben-Weyl, Methoden der organischen Chemie, Thieme-Verlag Stuttgart 1957, 4. Auflage, Band 11/1, S. 360 ff.) reduziert.

Die Ausgangsverbindungen der Formel III a wurden nach literaturbekannten Analogieverfahren (z. B. S. Takhashi, H. Kano, Chem. Pharm. Bull. 11 (11), 1375 ff.) hergestellt.

Die neuen Verbindungen der Formel I und ihre Salze, insbesondere jene, in welchen R eine Gruppe $SR^6$ bedeutet, besitzen wertvolle pharmakologische Eigenschaften.

Sie hemmen deutlich die Magensäuresekretion und weisen darüberhinaus eine ausgezeichnete Magen- und Darmschutzwirkung auf.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z. B. Ulcus ventriculi, Ulcus duodeni, Gastritis, hyperazider oder medikamentös bedingter Reizmagen) verstanden, die beispielsweise durch Mikroorganismen, Bakterientoxine, Medikamente (z. B. Antiphlogistika und Antirheumatika), Chemikalien (z. B. Ethanol), Magensäure oder Streßsituationen verursacht werden können.

Aufgrund ihrer ausgezeichneten Eigenschaften sind die substituierten Pyrido(1,2-c)imidazo(1,2-a)benzimidazole und ihre pharmakologisch verträglichen Salze für den Einsatz in der Human- und Veterinärmedizin hervorragend geeignet, wobei sie insbesondere zur Behandlung und Prophylaxe von Krankheiten des Magens und Darms und solcher Krankheiten, die auf einer überhöhten Magensäuresekretion beruhen, verwendet werden.

Die Erfindung betrifft daher weiter die erfindungsgemäßen Verbindungen der Formel I zur Anwendung bei der Behandlung und Prophylaxe der vorstehend genannten Krankheiten.

Ebenso umfaßt die Erfindung die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere Verbindungen der allgemeinen Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (=Wirkstoffe) entweder als solche, oder vorzugsweise in Kombination mit geeigneten pharmazeutischen Hilfsstoffen in Form

von Tabletten, Dragees, Kapseln, Suppositorien, Emulsionen, Suspensionen oder Lösungen eingesetzt, wobei der Wirkstoffgehalt vorteilhafterweise zwischen 0,1 und 95 % beträgt.

Welche Hilfsstoffe für die gewünschte Arzneimittelformulierungen geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können oral oder parenteral appliziert werden, wobei die orale Applikation bevorzugt ist.

Im allgemeinen hat es sich in der Humanmedizin als vorteilhaft erwiesen, den oder die Wirkstoffe bei oraler Gabe in einer Tagesdosis von etwa 0,01 bis etwa 20, vorzugsweise 0,05 bis 7, insbesondere 0,1 bis 2 mg/kg Körpergewicht, gegebenenfalls in Form mehrerer, vorzugsweise 1 bis 4 Einzelgaben zur Erzielung des gewünschten Ergebnisses zu verabreichen. Bei der Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der obengenannten Krankheiten eingesetzt werden, so können die pharmazeutischen Zubereitungen auch einen oder mehrere pharmakologisch aktive Bestandteile anderer Arzneimittelgruppen, wie Antactida, beispielsweise Aluminiumhydroxid, Magnesiumaluminat; Tranquilizer, wie Benzodiazepine, beispielsweise Diazepam; Spasmolytika, wie z. B. Bietamiverin, Camylofin; Anticholinergica, wie z. B. Oxyphencyclimin, Phencarbamid; Lokalanaesthetika, wie z. B. Tetracain,

Procain; gegebenenfalls auch Fermente, Vitamine oder Aminosäuren enthalten.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige alkoholische oder ölige Lösungen. Als inerte Träger können z. B. Gummi arabicum, Magnesiumcarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weiteren Hilfsstoffe in Lösung, Suspension oder Emulsion gebracht. Als Lösungsmittel für die neuen aktiven Verbindungen und die entsprechenden physiologisch verträglichen Salze kommen z. B. in Frage: Wasser, physiologische Kochsalzlösungen oder Alkohole, z. B. Ethanol, Propanol oder Glycerin, daneben auch Zuckerlösungen wie Glucose- oder Mannitlösungen, oder auch eine Mischung aus den verschiedenen genannten Lösungsmitteln.

Die nachfolgenden Beispiele sollen die erfindungsgemäßen Verfahrensweisen erläutern, ohne die Erfindung auf die hier stellvertretend genannten Substanzen zu beschränken.

**BEISPIELE**

1.) Pyrido(1,2-c)imidazo(1,2-a)benzimidazol-12-sulfenyl

a) 3 g 2-[(2-Pyridylmethyl)sulfinyl]benzimidazol werden in 300 ml 2 n HCl gelöst und 3 Std. bei Raumtemperatur stehengelassen. Anschließend wird mit wäßriger konz. $NH_3$-Lösung auf pH 7 eingestellt und der ausgefallene Niederschlag abfiltriert. Das gebildete, intensiv violett gefärbte Pyrido(1,2-c)imidazo(1,2-a)benzimidazol-12-sulfenyl kann durch Verreiben mit Essigester oder durch Säulenchromatographie an Kieselgel mit einem $CHCl_3$/Methanol-Gemisch (7 : 3) als Laufmittel gereinigt werden.

Fp: 190°C (Zers.)

b) 2 g Pyrido(1,2-c)imidazo(1,2-a)benzimidazol, zugänglich auf den unten beschriebenen Wegen, werden mit 0,4 g elementarem Schwefel gemischt und 15 Min. im Ölbad auf 150°C (Badtemperatur) erhitzt. Anschließend wird das Reaktionsprodukt in $CHCl_3$/Methanol (7 : 3) aufgenommen und säulenchromatographisch, wie unter 1 a) beschrieben, gereinigt. Das Produkt ist in jeder Beziehung identisch mit dem unter 1 a) beschriebenen.

c) 4,1 g Pyrido(1,2-c)imidazo(1,2-a)benzimidazol werden in 200 ml Tetrahydrofuran gelöst. Bei Raumtemperatur setzt man 0,8 ml Dischwefeldichlorid zu und rührt 2 Stunden nach. Nach Stehen über Nacht filtriert man ab und reinigt das Produkt wie unter 1 a) beschrieben. Die nach 1 a) - c) erhaltenen Produkte sind identisch.

2.) 12-Methylthio-pyrido(1,2-c)imidazo(1,2-a)-benzimidazol

2,4 g Pyrido-(1,2-c)imidazo(1,2-a)-benzimidazol-12-sulfenyl werden in 100 ml THF, dem man 10 ml Methanol zugesetzt hat, gelöst und mit 500 mg $NaBH_4$ unter Stickstoffatmosphäre bei Raumtemperatur reduziert zur 12-Mercaptoverbindung die ohne Zwischenisolierung mit Dimethylsulfat (2 ml) bei Raumtemperatur in die 12-Methylthioverbindung überführt wird. (Reaktionszeit 5 Minuten). Isolierung und Reinigung erfolgt durch Säulenchromatographie an Kieselgel mit Essigester als Laufmittel.

Fp: 119 - 121°C.

3.) 12-Acetylthio-pyrido(1,2-c)imidazo(1,2-a)-benzimidazol

2,4 g Pyrido-(1,2-c)imidazo(1,2-a)benzimidazol-12-sulfenyl werden, wie unter 2.) beschrieben, mit $NaBH_4$ reduziert und anschließend mit 2 ml Acetanhydrid bei Raumtemperatur in die 12-Acetylthioverbindung überführt. Isolierung und Reinigung erfolgt wie unter 2.) beschrieben.

Fp: 170°C (Zers.)

4) Pyrido(1,2-c)imidazo(1,2-a)benzimidazol

$a_1$) 1-(2-Picolyl)-2-chlor-benzimidazol

Zu einer Suspension von 41,9 g Natriumhydrid (55 - 60 %-ige Dispersion) in 500 ml Dimethylformamid werden bei 25°C zuerst 61,0 g 2-Chlorbenzimidazol und dann 66,0 g 2-Picolylchlorid Hydrochlorid zugefügt. Man rührt 1 Stunde bei Raumtemperatur und 2 Stunden bei 60°C nach. Nach Verdünnen mit 1 l Ethylacetat tropft man vorsichtig 250 ml Wasser zu, trennt die Phasen und engt die Essigester-Phase im Vakuum ein. Das zurückbleibende Produkt schmilzt bei 75 - 77°C.

$a_2$) Pyrido (1,2-c)imidazo(1,2-a)benzimidazol

Das obige Produkt wird in überschüssiger äthanolischer Salzsäure erwärmt (15 Minuten). Nach dem Abkühlen saugt man das Hydrochlorid ab (Fp: 258 - 260°C) und überführt es mit wäßriger Natronlauge in die Base, die durch Absaugen isoliert wird.

Fp: 163 - 165°C.

$b_1$) N-2-Picolyliden-o-nitroanilin

27,6 g c-Nitroanilin und 21,4 g 2-Pyridinaldehyd werden in 200 ml Toluol 10 Std. lang erhitzt, und das gebildete Wasser wird mit einem Wasserabscheider gesammelt. Anschließend wird die Lösung mit 500 ml Diethylether versetzt und die ausfallenden Kristalle abgesaugt.

Fp: 108 - 110°C

$b_2$) N-2-Picolyl-o-nitroanilin

Die obigen Kristalle (20 g) werden in 150 ml Ethanol gelöst und mit Hilfe von 10 g $NaBH_4$ bei der Siedetemperatur des Alkohols innerhalb einer Stunde reduziert. Nach Zugabe von 20 ml Wasser zur Zersetzung des $NaBH_4$ wird der Alkohol im Vakuum eingedampft und das Reduktionsprodukt abfiltriert.

Fp: 86 - 88°C.

$b_3$) N-2-Picolyl-o-phenylendiamin

10 g N-2-Picolyl-o-nitroanilin werden mit Raney-Nickel in Methanol hydriert bei Raumtemperatur zum o-Phenylendiamin-Derivat, das nach Abfiltrieren des Katalysators und Eindampfen des Lösungsmittels im Vakuum als Öl zurückbleibt.

$b_4$) 1-(2-Pyridylmethyl)benzimidazolin-2-on

5 g des obigen o-Phenylendiamin-Derivates werden mit 2 g Harnstoff 5 Std. auf 160°C erhitzt. Nach Abkühlen der Reaktion gibt man Ethanol hinzu und saugt die ausgefallenen Kristalle ab.

Fp: 168 - 170°C

$b_5$) Pyrido(1,2-c)imidazo(1,2-a)benzimidazol

2 g des obigen 1-(2-Pyridylmethyl)-benzimid-azolin-2-on werden mit 10 ml $POCl_3$ 5 Std. lang am Rückfluß gekocht. Danach wird das überschüssige $POCl_3$ im Vakuum abgezogen, der Rückstand in Wasser gegeben, mit NaOH neutralisiert und extrahiert mit $CH_2Cl_2$. Nach Trocknen mit $Na_2SO_4$ und Eindampfen des Lösungsmittels hinterbleibt das Zyklisierungsprodukt, das in jeder Beziehung identisch ist mit der unter 4 a) beschriebenen Verbindung.

**Tabelle 1**

Weiter hergestellte Verbindungen der Formel I ($R^3$ bis $R^5$ jeweils = Wasserstoff):

| Beispiel Nr. | $R^1$ | $R^2$ | R | Fp. | Herstellungsverf. analog Beispiel |
|---|---|---|---|---|---|
| *) 5 | $CH_3$, H | H, $CH_3$ | S. | 126°C | 1a) |
| *) 6 | $Bu^t$, H | H, $Bu^t$ | S. | 248°C | 1 a) |
| 7 | H | H | $SC_2H_5$ | 126 - 127°C | 2) |
| 8 | H | H | $SCH_2$-$C_6H_5$ | 163°C (Zers.) | 2) |
| 9 | H | H | $SCH_2$-C≡CH | 156°C (Zers.) | 2) |
| 10 | H | H | $SCH_2$-CH=$CH_2$ | 94 - 95°C | 2) |
| 11 | H | H | SCO-$C_2H_5$ | 143 - 145°C | 3) |
| 12 | H | H | $SCO(CH_2)_2CH_3$ | 138°C | 3) |
| 13 | H | H | $SCO(CH_2)_5CH_3$ | 141°C | 3) |
| 14 | H | H | SCO-$C_6H_5$ | 196 - 198°C | 3) |
| 15 | H | $Bu^t$ | $SCOCH_3$ | 135°C | 3) **) |

| Beispiel Nr. | $R^1$ | $R^2$ | R | Fp. | Herstellungsverf. analog Beispiel |
|---|---|---|---|---|---|
| 16 | $Bu^t$ | H | $SCOCH_3$ | 130°C | 3) **) |
| 17 | H | H | $SCOC(CH_3)_3$ | 162°C | 3) |
| 18 | $OCH_3$ | $OCH_3$ | $SCOOH_3$ | 181°C (Zers.) | 3) |
| 19 | $O\text{-}(CH_2)_2CH_3$ | $O\text{-}(CH_2)_2CH_3$ | $SCOOH_3$ | 132°C (Zers.) | 3) |
| 20 | H | H | $SCH_2O(CH_2)OCH_3$ | 96 - 97°C | 2) |
| 21 | H | H | $SCH_2\text{-}CH=CH_2$ | 121 - 122°C | 2) |
| 22 | H | $OCH_3$ | $SCO\text{-}OC(CH_3)_3$ | 149°C | 3) |
| 23 | H | H | $SCOCH(C_2H_5)_2$ | 150 - 152°C | 3) |
| 24 | H | H | S-CO- 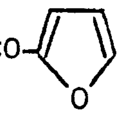 | 200 - 201°C | 3) |
| 25 | H | H | $SCO\text{-}CH_2\text{-}C_6H_5$ | 175 - 177°C | 3) |
| 26 | H | H | $SCOCH_2\text{-}O\text{-}$ 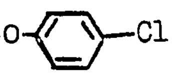 | 162°C | 3) |
| 27 | H | H | SCO-Cyclohexyl | 176°C | 3) |
| 28 | H | H | $SCO\text{-}CH=CH\text{-}CH_3$ | 155°C | 3) |
| 29 | H | H | $SCOCH_2\text{-}OCH_3$ | 160°C | 3) |
| 30 | H | H | $SCOCH_2\text{-}$ 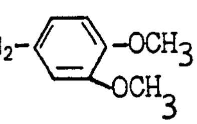 | 160 - 162°C | 3) |
| 31 | H | H | $SCOH_2\text{-}OC_6H_5$ | 167°C | 3) |
| 32 | H | $C(CH_3)_3$ | $SCOCH_3$ | 135°C | 3) |
| 33 | $C(CH_3)_3$ | H | $SCOCH_3$ | 130°C | 3) |
| 34 | H | $CO\text{-}OC_2H_5$ | $SCOCH_3$ | 128 - 129°C | 3) |
| 35 | H | $OCH_3$ | $SCO\text{-}OC(CH_3)_3$ | 160°C (Zers.) | 3) |
| *36 | $H, CO\text{-}OC_2H_5$ | $H, CO\text{-}OC_2H_5$ | S. | 154 - 156°C | 1a) |
| 37 | Cl | H | S. | 197°C (Zers.) | 1b) |

*) Isomergemische

**) Nach säulenchromatischer Trennung an Kieselgel (Grace[R] 50) mit Essigester als Elutionsmittel.

$Bu^t = \text{-}C(CH_3)_3$

**Tabelle 2**

Weiter hergestellte Verbindungen der Formel I
$R^1$ bis $R^5$ jeweils = Wasserstoff,

R = 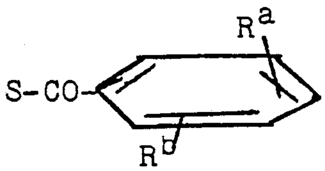 )

| Beispiel Nr | $R^a$ | $R^b$ | Fp. | Herstellungsverf. analog Beispiel |
|---|---|---|---|---|
| 38 | 4-$CF_3$ | H | 180°C | 3) |
| 39 | 3-Cl | H | 180°C | 3) |
| 40 | 4-$C(CH_3)_3$ | H | 211°C | 3) |
| 41 | 3-$OCH_3$ | 5-$OCH_3$ | 190°C | 3) |
| 42 | 4-$CH_3$ | H | 204°C | 3) |
| 43 | 4-Cl | H | 174°C | 3) |
| 44 | 3-$CH_3$ | H | 170°C | 3) |
| 45 | 2-$CH_3$ | H | 195°C | 3) |
| 46 | 2-Cl | H | 164°C | 3) |
| 47 | 4-$OCH_3$ | H | 190 - 192°C | 3) |
| 48 | 4-$OC(CH_3)_3$ | H | 205°C | 3) |
| 49 | 4-CN | H | 184 - 186°C | 3) |
| 50 | 4-$(CH_2)_5CH_3$ | H | 146°C | 3) |
| 51 | 4-$(CH_2)_6CH_3$ | H | 182 - 183°C | 3) |
| 52 | 4-Cl | 3-Cl | 157°C | 3) |
| 53 | 4-$(CH_2)_4CH_3$ | H | 182 - 183°C | 3) |
| 54 | 4-O-$(CH_2)_4CH_3$ | H | 169°C | 3) |
| 55 | 3-$OCH_3$ | H | 184°C | 3) |
| 56 | 2-Cl | 4-Cl | 180°C | 3) |
| 57 | 2-Cl | 6-$CH_3$ | | 3) |
| 58 | 3-$CH_3$ | 5-$CH_3$ | | 3) |
| 59 | R = 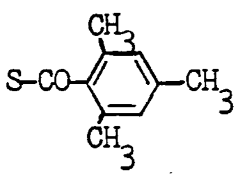 | | | 3) |

60.) <u>2-Methoxy-1,3-dimethyl-pyrido[1,2-c]imidazo[1,2-a]benzimidazol-12-sulfenyl</u>

2 g 1-(4-Methoxy-3,5-dimethyl-2-picolyl)benzimidazol-3-oxid werden in 100 ml $CH_2Cl_2$ gelöst, anschließend wird bei Raumtemperatur 1 ml Trifluoracetanhydrid zugetropft und die Lösung 1 Std. bei Raumtemperatur gerührt. Anschließend wird die Reaktionsmischung eingedampft und der Rückstand mit Isopropanol gewaschen.

Ausbeute: 2 g

Fp: 300°C

Das so hergestellte 2-Methoxy-1,3-dimethyl-pyrido[1,2-c]-imidazo[1,2-a]benzimidazol-trifluoracetat wird mit 1 n NaOH in die freie Base überführt, mit $CH_2Cl_2$ extrahiert, über $Na_2SO_4$ getrocknet und einrotiert. Das zurückbleibende gelbe Öl wird, wie bei Beispiel 1 b beschrieben, durch Reaktion mit Schwefel der Titelverbindung überführt.

Ausbeute: 1,5 g

Schmelzpunkt: 260°C (Zers.)

**Beispiel 61.)**

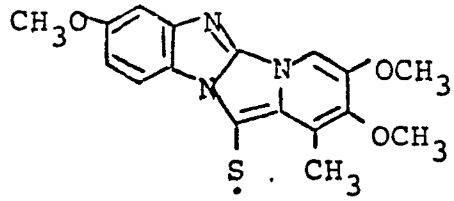

Herstellung analog Beispiel 60.
Fp: 196°C

**Patentansprüche** für die Vertragsstaaten: BE, CH, DE, FR, GB, IT, LI, LU, NL, SE

1. Verbindung der Formel I

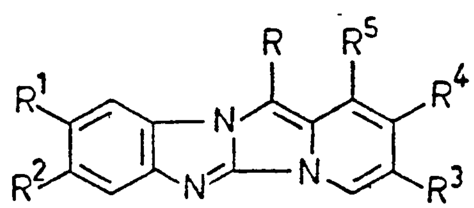

(I)

in welcher
$R^1$ und $R^2$ gleich oder verschieden sind und Wasserstoff, ($C_1$ bis $C_6$)-Alkyl, Trifluormethyl, Halogen, Methoxycarbonyl, Ethoxycarbonyl, ($C_1$ bis $C_5$)-Alkoxy oder ($C_1$ bis $C_4$)-Alkanoyl bedeuten,
$R^3$, $R^4$ und $R^5$ gleich oder verschieden sind und Wasserstoff, Methyl, Methoxy, Ethoxy, Methoxyethoxy oder Ethoxyethoxy bedeuten und
R Wasserstoff oder eine Gruppe $SR^6$ bedeutet, worin $R^6$ für das freie, ungepaarte Elektron des 12-Sulfenylradikals, Wasserstoff, ($C_1$ bis $C_6$)-Alkyl, das gegebenenfalls mit ($C_1$ bis $C_4$)-Alkoxy oder ein- oder mehrfach mit ($C_6$ bis $C_{10}$)-Aryl substituiert sein kann, ($C_2$ bis $C_6$)-Alkenyl, ($C_2$ bis $C_6$)-Alkinyl, ($C_1$ bis $C_7$)-Alkanoyl, ($C_5$ bis $C_7$)-Cycloalkanoyl, Benzoyl, das gegebenenfalls ein- oder mehrfach mit ($C_1$ bis $C_7$)-Alkyl, ($C_1$ bis $C_5$)-Alkoxy, ($C_1$ bis $C_4$)-Alkoxycarbonyl, Cyano, Trifluormethyl und/oder Halogen substituiert ist. Furoyl, Phenylacyl, Phenoxyacetyl, Phenylacetyl, wobei die drei letzteren wie Benzoyl substituiert sein können, oder eine andere gebräuchliche Thiol-Schutzgruppe steht, sowie deren physiologisch verträglichen Salze.

2. Verfahren zur Herstellung von Verbindungen der Formel I gemäß Anspruch 1, dadurch gekennzeichnet, daß man
$a_1$) zur Herstellung von Verbindungen der Formel I mit R = Wasserstoff
Verbindungen der Formel II,

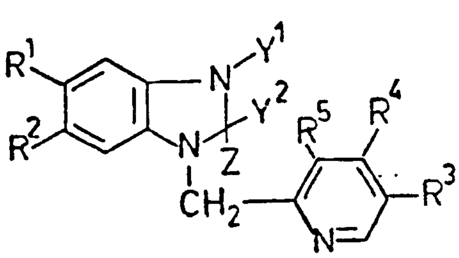

(II)

in welcher $R^1$ bis $R^5$ wie im Anspruch 1 definiert sind und entweder $Y^1$ + $Y^2$ zusammen für eine Bindung und Z für eine Abgangsgruppe oder $Y^1$ für Wasserstoff und $Y^2$ + Z zusammen für eine Oxogruppe stehen,
cyclisiert,
$a_2$) Verbindungen der Formel III a

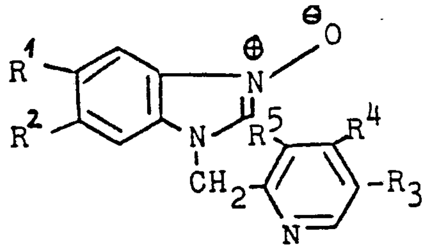

(III a)

in welcher $R^1$ bis $R^5$ wie oben definiert sind, cyclisiert,

b) zur Herstellung von Verbindungen der Formel I mit R = Sulfenyl

$b_1$) 2-Benzimidazol-2-pyridyl-sulfoxide der Formel III

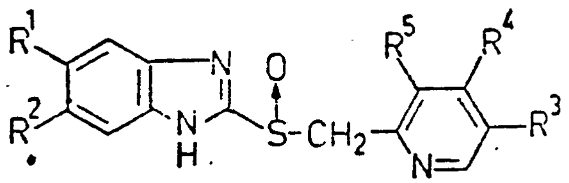

(III)

in welcher $R^1$ bis $R^5$ wie oben definiert sind, mit einer Säure behandelt, wobei, im Falle $R^1 \neq R^2$ Gemische stellungsisomerer Verbindungen der Formel I entstehen können, oder

$b_2$) Verbindungen der Formel I, in welcher $R^1$ bis $R^5$ wie oben definiert sind und R Wasserstoff bedeutet, mit elementarem Schwefel oder mit $S_2Cl_2$ in einem organischen Lösungsmittel umsetzt oder

c) zur Herstellung von Verbindungen der Formel I

R = Wasserstoff oder Sulfenyl

Verbindungen der Formel I, in welcher $R^1$ bis $R^5$ wie oben definiert sind und R Sulfenyl bedeutet, reduziert zu Verbindungen der Formel I, worin $R^6$ Wasserstoff ist und diese gewünschtenfalls umsetzt mit Verbindungen der Formel $R^6X$, worin $R^6$ im Anspruch 1 definierten Bedeutungen außer Wasserstoff hat und X eine Abgangsgruppe ist,

und die nach $a_1$) - c) erhaltenen Verbindungen gegebenenfalls in ihre physiologisch verträglichen Salze überführt.

3. Mittel enthaltend eine Verbindung der Formel I gemäß Anspruch 1.

4. Mittel enthaltend zwei oder mehrere Verbindungen der Formel I gemäß Anspruch 1.

5. Mittel gemäß Anspruch 3 oder 4, dadurch gekennzeichnet, daß es einen pharmazeutisch unbedenklichen Träger und gegebenenfalls weitere Hilfsstoffe, wie Stabilisatoren enthält.

6. Verbindung gemäss Anspruch 1 oder Mittel gemäß einem der Ansprüchs 3 bis 5 zur Anwendung als Heilmittel.

7. Verwendung oder Mittel gemäß Anspruch 6 zur Anwendung als Magensäuresekretionshemmer.

8. Verbindung der Formel II, in der $R^1$ bis $R^5$, $Y^1$, $Y^2$ und Z wie im Anspruch 2 definiert sind.

9. Verfahren zur Herstellung von Verbindungen der Formel II gemäß Anspruch 8, dadurch gekennzeichnet, daß man

a) zur Herstellung einer Verbindung der Formel II mit $Y^1 + Y^2$ = Bindung und Z = Abgangsgruppe eine Verbindung der Formel IV

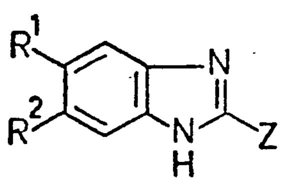

(IV)

in welcher $R^1$ und $R^2$ wie im Anspruch 1 und Z wie oben definiert sind, umsetzt mit einem substituierten Picolylderivat der Formel V

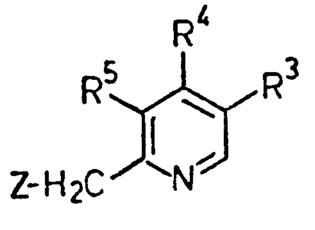

(V)

in welcher $R^3$ bis $R^5$ wie im Anspruch 1 und Z wie oben definiert sind oder

b) zur Herstellung einer Verbindung der Formel mit $Y^1$ = Wasserstoff und $Y^2 + Z$ = Oxo eine Verbindung der Formel VI

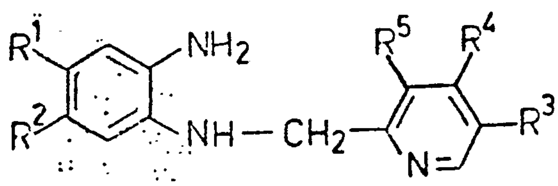

(VI)

in welcher $R^1$ bis $R^5$ wie oben definiert sind, mit einem Kohlensäurederivat umsetzt.

10. Verfahren zur Herstellung eines Mittels gemäß einem der Ansprüche 3 bis 5, dadurch gekennzeichnet, daß man Verbindungen der Formel I in eine geeignete Darreichungsform bringt.

**Patentansprüche** für den Vertragsstaat Österreich

1. Verfahren zur Herstellung von Verbindungen der Formel I

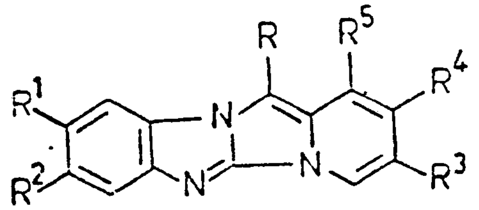

(I)

in welcher

$R^1$ und $R^2$ gleich oder verschieden sind und Wasserstoff, ($C_1$ bis $C_6$)-Alkyl, Trifluormethyl, Halogen, Methoxycarbonyl, Ethoxycarbonyl ($C_1$ bis $C_5$)-Alkoxy oder ($C_1$ bis $C_4$)-Alkanoyl bedeuten,

$R^3$, $R^4$ und $R^5$ gleich oder verschieden sind und Wasserstoff, Methyl, Methoxy, Ethoxy, Methoxyethoxy oder Ethoxyethoxy bedeuten

und

R Wasserstoff oder eine Gruppe $SR^6$ bedeutet, worin $R^6$ für das freie, ungepaarte Elektron des 12-Sulfenylradikals, Wasserstoff, ($C_1$ bis $C_6$)-Alkyl, das gegebenenfalls mit ($C_1$ bis $C_4$)-Alkoxy oder ein- oder mehrfach mit ($C_6$ bis $C_{10}$)-Aryl substituiert sein kann, ($C_2$ bis $C_6$)-Alkenyl, ($C_2$ bis $C_6$)-Alkinyl, ($C_1$ bis $C_7$)-Alkanoyl, ($C_5$ bis $C_7$)-Cycloalkanoyl, Benzoyl, das gegebenenfalls ein- oder mehrfach mit ($C_1$ bis $C_7$)-Alkyl, ($C_1$ bis $C_5$)-Alkoxy, ($C_1$ bis $C_4$)-Alkoxycarbonyl, Cyano, Trifluormethyl und/oder Halogen substituiert ist, Furoyl, Phenylacyl oder eine andere gebräuchliche Thiol-Schutzgruppe steht,

sowie deren physiologisch verträglichen Salzen, dadurch gekennzeichnet, daß man

$a_1$) zur Herstellung von Verbindungen der Formel I mit R = Wasserstoff

Verbindungen der Formel II,

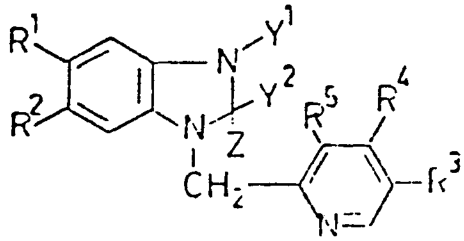

(II)

in welcher $R^1$ bis $R^5$ wie im Anspruch 1 definiert sind und entweder $Y^1 + Y^2$ zusammen für eine Bindung und $Y^1$ für Wasserstoff und $Y^2 + Z$ zusammen für eine Oxogruppe stehen,
cyclisiert,
$a_2$) Verbindungen der Formel III a

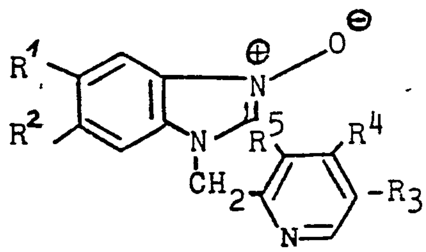

(III a)

in welcher $R^1$ bis $R^5$ wie oben definiert sind, cyclisiert,
b) zur Herstellung von Verbindungen der Formel I mit R = Sulfenyl
$b_1$) 2-Benzimidazol-2-pyridyl-sulfoxide der Formel III

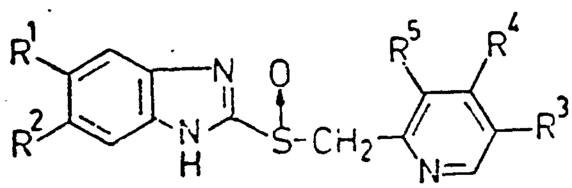

(III)

in welcher $R^1$ bis $R^5$ wie oben definiert sind, mit einer Säure behandelt, wobei im Falle $R^1 + R^2$ Gemische stellungsisomerer Verbindungen der Formel I entstehen können, oder

$b_2$) Verbindungen der Formel I in welcher $R^1$ bis $R^5$ wie oben definiert sind und R Wasserstoff bedeutet, mit elementarem Schwefel oder mit $S_2Cl_2$ in einem organischen Lösungsmittel umsetzt oder

c) zur Herstellung von Verbindungen der Formel I R = Wasserstoff oder Sulfenyl Verbindungen der Formel I, in welcher $R^1$ bis $R^5$ wie oben definiert sind und R Sulfenyl bedeutet, reduziert zu Verbindungen der Formel I, worin $R^6$ Wasserstoff ist und diese gewünschtenfalls umsetzt mit Verbindungen der Formel $R^6X$, worin $R^6$ im Anspruch 1 definierten Bedeutungen außer Wasserstoff hat und X eine Abgangsgruppe ist,

und die nach $a_1$) - c) erhaltenen Verbindungen gegebenenfalls in ihre physiologisch verträglichen Salze überführt.

2. Verfahren zur Herstellung eines Mittels enthaltend eine Verbindung der Formel I gemäß Anspruch 1, dadurch gekennzeichnet, daß man diese in eine geeignete Darreichungsform bringt.

3. Verfahren gemäß Anspruch 2, dadurch gekennzeichnet, daß ein Mittel aus zwei oder mehreren Verbindungen der Formel I hergestellt wird.

4. Verfahren gemäß Anspruch 2 oder 3, dadurch gekennzeichnet, daß man ein Mittel herstellt, das einen pharmazeutisch unbedenklichen Träger und gegebenenfalls weitere Hilfsstoffe, wie Stabilisatoren enthält.

5. Verfahren gemäß Anspruch 1 zur Herstellung einer Verbindung zur Anwendung als Heilmittel.

6. Verfahren gemäß Anspruch 1 zur Herstellung einer Verbindung zur Anwendung als Magensäuresekretionshemmer.

7. Verfahren zur Herstellung von Verbindungen der Formel II in der $R^1$ bis $R^5$, $Y^1$, $Y^2$ und Z wie im Anspruch definiert sind, dadurch gekennzeichnet, daß man

a) zur Herstellung einer Verbindung der Formel II mit $Y^1 + Y^2$ Bindung und Z= Abgangsgruppe eine Verbindung der Formel IV

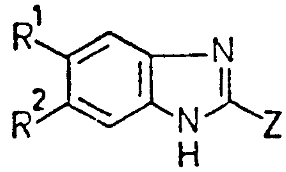

(IV)

in welcher $R^1$ und $R^2$ wie im Anspruch 1 und Z wie oben definiert sind, umsetzt mit einem substituierten Picolylderivat der Formel V

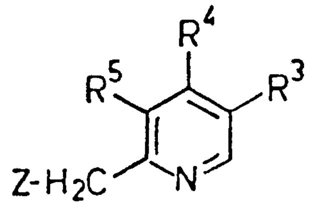

(V)

in welcher $R^3$ bis $R^5$ wie im Anspruch 1 und Z wie oben definiert sind oder

b) zur Herstellung einer Verbindung der Formel II mit $Y^1$ = Wasserstoff und $Y^2 + Z$ = Oxo eine Verbindung der Formel VI

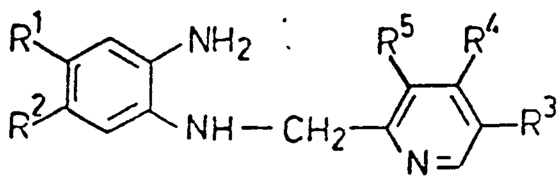

(VI)

in welcher $R^1$ bis $R^5$ wie oben definiert sind, mit einem Kohlensäurederivat umsetzt.

**Claims** : for the contracting states: BE, CH, DE, FR, GB, IT, LI, LU, NL, SE

1. A compound of the formula I

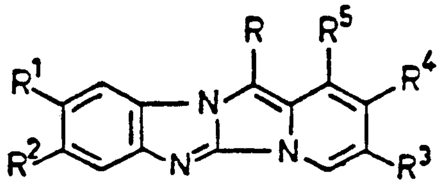

(I)

in which $R^1$ and $R^2$ are identical or different and are hydrogen, ($C_1$ to $C_6$)-alkyl, trifluoromethyl, halogen, methoxycarbonyl, ethoxycarbonyl, ($C_1$ to $C_5$)-alkoxy or ($C_1$ to $C_4$)-alkanoyl,

$R^3$, $R^4$ and $R^5$ are identical or different and are hydrogen, methyl, methoxy, ethoxy, methoxyethoxy or ethoxyethoxy and

R is hydrogen or a group $SR^6$, in which $R^6$ is the lone, unpaired electron of the 12-sulfenyl radical, hydrogen, ($C_1$ to $C_6$)-alkyl which can optionally be substituted by ($C_1$ to $C_4$)-alkoxy or mono- or poly-substituted by ($C_6$ to $C_{10}$)-aryl or is ($C_2$ to $C_6$)-alkenyl, ($C_2$ to $C_6$)-alkynyl, ($C_1$ to $C_7$)-alkanoyl, ($C_5$ to $C_7$)-cycloalkanoyl, benzoyl which is optionally mono- or poly-substituted by ($C_1$ to $C_7$)-alkyl, ($C_1$ to $C_5$)-alkoxy, ($C_1$to $C_4$)-alkoxycarbonyl, cyano, trifluoromethyl and/or halogen, or is furoyl, phenylacyl, phenoxyacetyl, phenylacetyl, it being possible for the latter three to be substituted in the same way as benzoyl, or another conventional thiol-protective group, and physiologically acceptable salts thereof.

2. A process for the preparation of a compound of the formula I as claimed in Claim 1, which comprises,

$a_1$) for the preparation of a compound of the formula I with R = hydrogen, cyclizing a compound of the formula II

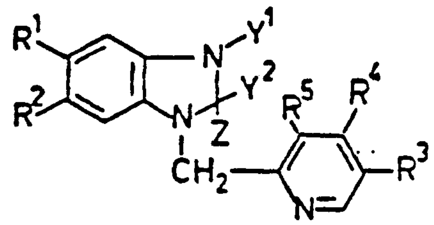

(II)

in which $R^1$ to $R^5$ are as defined in claim 1 and either $Y^1 + Y^2$ together represent a bond and Z represents a leaving group or $Y^1$ is hydrogen and $Y^2 + Z$ together represent an oxo group,

$a_2$) cyclizing a compound of the formula III a

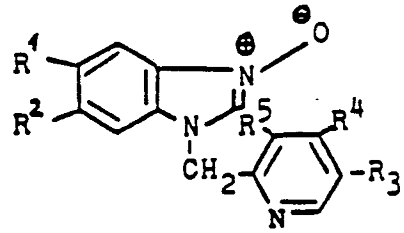

(III a)

in which $R^1$ to $R^5$ are as defined above,

b) for the preparation of a compound of the formula I with R = sulfenyl,

$b_1$) treating a 2-benzimidazole 2-pyridyl sulfoxide of the formula III

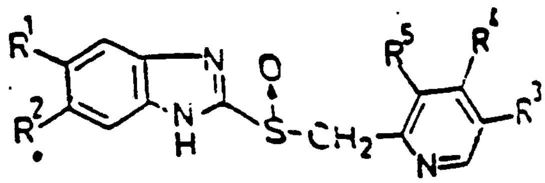

(III)

in which $R^1$ to $R^5$ are as defined above, with an acid, in which case mixtures of positionally isomeric compounds of the formula I can be formed if $R^1 \neq R^2$, or

$b_2$) reacting a compound of the formula I, in which $R^1$ to $R^5$ are as defined above and R is hydrogen, with elemental sulfur or with $S_2Cl_2$ in an organic solvent, or

c) for the preparation of a compound of the formula I with R = hydrogen or sulfenyl, reducing the compound of the formula I, in which $R^1$ to $R^5$ are as defined above and

R is sulfenyl, to a compound of the formula I, in which $R^6$ is hydrogen, and, if desired, reacting the latter compound with a compound of the formula $R^6X$, in which $R^6$ has one of the meanings defined in claim 1 with the exception of hydrogen and X is a leaving group, and optionally converting the compounds obtained according to $a_1$) - c) into their physiologically acceptable salts.

3. An agent which contains a compound of the formula I, as claimed in claim 1.

4. An agent which contains two or more compounds of the formula I, as claimed in claim 1.

5. The agent as claimed in claim 3 or 4, which contains a pharmaceutically acceptable carrier and, if appropriate, further auxiliaries, such as stabilizers.

6. A compound as claimed in claim 1 or an agent as claimed in any of claims 3 to 5 for use as a medicament.

7. A compound or agent as claimed in claim 6 for use as a gastric acid secretion inhibitor.

8. A compound of the formula II, in which $R^1$ to $R^5$, $Y^1$, $Y^2$ and Z are as defined in claim 2.

9. A process for the preparation of a compound of the formula II as claimed in claim 8, which comprises,

a) for the preparation of a compound of the formula II with $Y^1 + Y^2$ = a bond and Z = a leaving group, reacting a compound of the formula IV

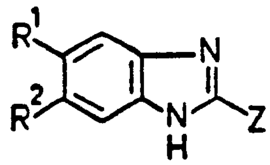

(IV)

in which $R^1$ and $R^2$ are as defined in Claim 1 and Z is as defined above, with a substituted picolyl derivative of the formula V

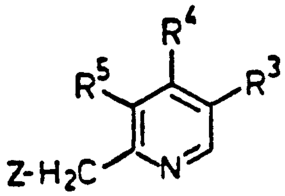

(V)

in which $R^3$ to $R^5$ are as defined in claim 1 and Z is as defined above, or

b) for the preparation of a compound of the formula with $Y^1$ = hydrogen and $Y^2 + Z$ = oxo, reacting a compound of the formula VI

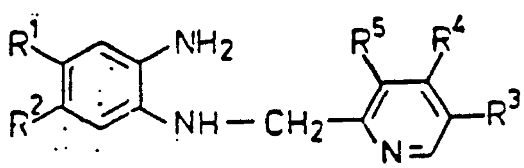

(VI)

in which $R^1$ to $R^5$ are as defined above, with a carbonic acid derivative.

10. The process for the preparation of an agent as claimed in one of claims 3 to 5, which comprises bringing compounds of the formula I into a suitable form for administration.

**Claims** : for the contracting state AT

1. A process for the preparation of a compound of the formula I

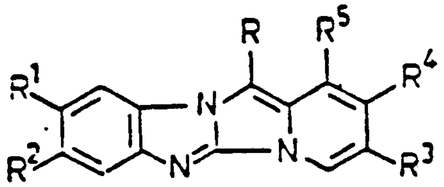

(I)

in which

$R^1$ and $R^2$ are identical or different and are hydrogen, ($C_1$ to $C_6$)-alkyl, trifluoromethyl, halogen, methoxycarbonyl, ethoxycarbonyl, ($C_1$ to $C_5$)-alkoxy or ($C_1$ to $C_4$)-alkanoyl,

$R^3$, $R^4$ and $R^5$ are identical or different and are hydrogen, methyl, methoxy, ethoxy, methoxyethoxy or ethoxyethoxy and

R is hydrogen or a group $SR^6$, in which $R^6$ is the lone, unpaired electron of the 12-sulfenyl radical, hydrogen, ($C_1$ to $C_6$)-alkyl which can optionally be substituted by ($C_1$ to $C_4$)-alkoxy or mono- or poly-substituted by ($C_6$ to $C_{10}$)-aryl, or is ($C_2$ to $C_6$)-alkenyl, ($C_2$ to $C_6$)-alkynyl, ($C_1$ to $C_7$)-alkanoyl, ($C_5$ to $C_7$)-cycloalkanoyl, benzoyl which is optionally mono- or poly-substituted by ($C_1$ to $C_7$)-alkyl, ($C_1$ to $C_5$)-alkoxy, ($C_1$ to $C_4$)-alkoxycarbonyl, cyano, trifluoromethyl and/or halogen, or is furoyl, phenylacyl or another conventional thiol-protective group, and physiologically acceptable salts thereof, which comprises,

$a_1$) for the preparation of a compound of the formula I

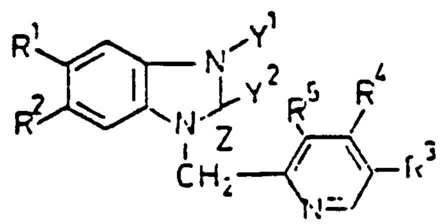

(II)

in which $R^1$ to $R^5$ are as defined in claim 1 and either $Y^1 + Y^2$ together represent a bond and Z represents a leaving group or $Y^1$ is hydrogen and $Y^2 + Z$ together represent an oxo group,

$a_2$) cyclizing a compound of the formula III a

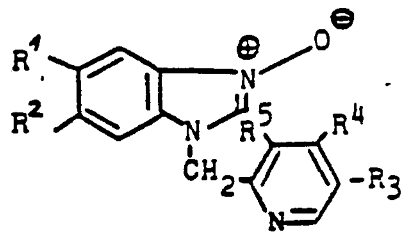

(III a)

in which $R^1$ to $R^5$ are as defined above,

b) for the preparation of a compound of the formula I with R = sulfenyl,

$b_1$) treating a 2-benzimidazole 2-pyridyl sulfoxide of the formula III

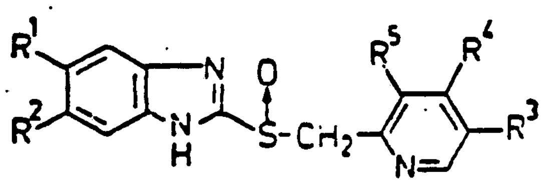

(III)

in which $R^1$ to $R^5$ are as defined above, with an acid, in which case mixtures of positionally isomeric compounds of the formula I can be formed if $R^1 + R^2$, or

$b_2$) reacting a compound of the formula I, in which $R^1$ to $R^5$ are as defined above and R is hydrogen, with elemental sulfur or with $S_2Cl_2$ in an organic solvent, or

c) for the preparation of a compound of the formula I with R = hydrogen or sulfenyl, reducing a compound of the formula I, in which $R^1$ to $R^5$ are as defined above and R is sulfenyl, to a compound of the formula I, in which $R^6$ is hydrogen, and, if desired, reacting the latter compound with a compound of the formula $R^6x$, in which $R^6$ has one of the meanings defined in claim 1 with the exception of hydrogen and X is a leaving group, and

optionally converting the compounds obtained according to $a_1$) - c) into their physiologically acceptable salts.

2. A process for the preparation of an agent which contains a compound of the formula I, as claimed in claim 1, which comprises bringing a compound of the formula I into a suitable form for administration.

3. The process as claimed in claim 2, wherein an agent is prepared from two or more compounds of the formula I.

4. The process as claimed in claim 2 or 3, wherein an agent is prepared which contains a pharmaceutically acceptable carrier and, if appropriate, further auxiliaries, such as stabilizers.

5. The process as claimed in claim 1 for the preparation of a compound for use as a medicament.

6. The process as claimed in claim 1 for the preparation of a compound for use as a gastric acid secretion inhibitor.

7. A process for the preparation of a compound of the formula II, in which $R^1$ to $R^5$, $Y^1$, $Y^2$ and Z are as defined in claim 1 which comprises,

a) for the preparation of a compound of the formula II with $Y^1 + Y^2$ = a bond and Z = a leaving group, reacting a compound of the formula IV

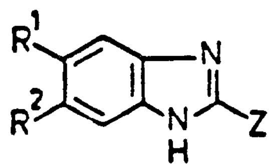

(IV)

in which $R^1$ and $R^2$ are as defined in claim 1 and Z is as defined above, with a substituted picolyl derivative of the formula V

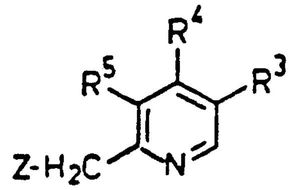

(V)

in which $R^3$ to $R^5$ are as defined in claim 1 and Z is as defined above, or

b) for the preparation of a compound of the formula II with $Y^1$ = hydrogen and $Y^2 + Z$ = oxo, reacting a compound of the formula VI

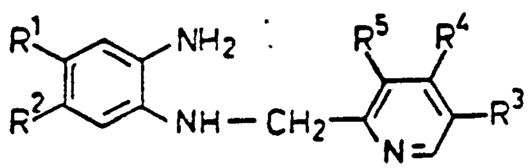

(VI)

in which $R^1$ to $R^5$ are as defined above, with a carbonic acid derivative.

**Revendications** Pour les Etats Contractants BE CH DE FR GB IT LI LU NL SE

1. Composé de formule I

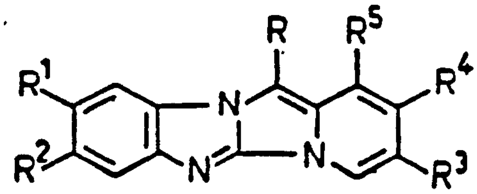

(I)

dans laquelle

$R^1$ et $R^2$ sont identiques ou différents, et représentent un atome d'hydrogène ou d'halogène ou un groupe alkyle en $C_1$-$C_6$, trifluorométhyle, méthoxycarbonyle, éthoxycarbonyle, alcoxy en $C_1$-$C_5$ ou alcanoyle en $C_1$-$C_4$;

$R^3$, $R^4$ et $R^5$ sont identiques ou différents, et représentent un atome d'hydrogène ou un groupe méthyle, méthoxy, éthoxy, méthoxyéthoxy ou éthoxyéthoxy; et

R représente un atome d'hydrogène ou un groupe $SR^6$, dans lequel $R^6$ représente l'électron non apparié du radical 12-sulfényle, un atome d'hydrogène, un groupe alkyle en $C_1$-$C_6$ qui peut éventuellement être substitué par un radical alcoxy en $C_1$-$C_4$ ou une ou plusieurs fois par un radical aryle en $C_6$-$C_{10}$, un groupe alcényle en $C_2$-$C_6$, alcinyle en $C_2$-$C_6$, alcanoyle en $C_1$-$C_7$, cycloalcanoyle en $C_5$-$C_7$, un groupe benzoyle qui peut éventuellement être substitué une ou plusieurs fois par un ou des radicaux alkyle en $C_1$-$C_7$, alcoxy en $C_1$-$C_5$, alcoxy($C_1$-$C_4$)-carbonyle, cyano, trifluorométhyle et/ou par des atomes d'halogène, un groupe furoyle, phénacyle, phénoxyacétyle, phénylacétyle, les trois derniers pouvant être substitués comme le groupe benzoyle, ou un autre groupe protecteur usuel de fonction thiol;

et sels physiologiquement acceptables de celui-ci.

2. Procédé pour la préparation de composés de formule I selon la revendication 1, caractérisé en ce que

a) pour la préparation de composés de formule I, dans lesquels R est un atome d'hydrogène,

$a_1$) on cyclise des composés de formule II

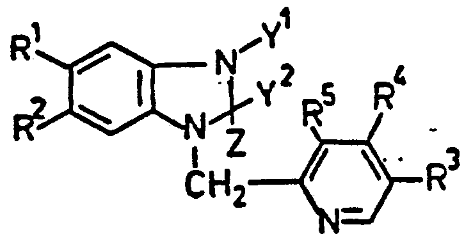

(II)

dans laquelle $R^1$ à $R^5$ sont tels que définis dans la revendication 1, et soit $Y^1 + Y^2$ représentent ensemble une liaison et Z représente un groupe séparable, soit $Y^1$ représente un atome d'hydrogène et $Y^2 + Z$ forment ensemble un groupe oxo;

$a_2$) on cyclise des composés de formule IIIa

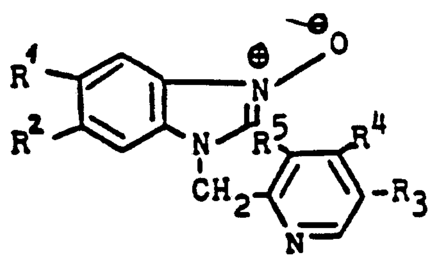

(IIIa)

dans laquelle $R^1$ à $R^5$ sont tels que définis plus haut;

b) pour la préparation de composés de formule I, dans lesquels R est le groupe sulfényle,

$b_1$) on traite par un acide un 2-benzimidazole-2'-pyridyl-sulfoxyde de formule III

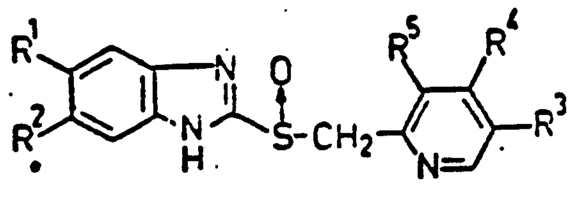

(III)

dans laquelle $R^1$ à $R^5$ sont tels que définis plus haut, ce par quoi, dans le cas où $R^1 \neq R^2$, il peut se former des mélanges de composés isomères de formule I, à isomérie de position; ou

$b_2$) on fait réagir avec du soufre élémentaire ou avec $S_2Cl_2$, dans un solvant organique, des composés de formule I dans lesquels $R^1$ à $R^5$ sont tels que définis plus haut et R représente un atome d'hydrogène;

c) pour la préparation de composés de formule I, dans lesquels R est un atome d'hydrogène ou le groupe sulfényle, on réduit des composés de formule I dans lesquels $R^1$ à $R^5$ sont tels que définis plus haut, et R représente le groupe sulfényle, pour aboutir aux composés de formule I dans lesquels $R^6$ est un atome d'hydrogène, et si on le désire, on fait réagir ceux-ci avec des composés de formule $R^6X$, dans lesquels $R^6$ a la signification donnée dans la revendication 1, à l'exception d'un atome d'hydrogène, et X est un groupe séparable;

et éventuellement on convertit les composés obtenus selon $a_1$) à c) en leurs sels physiologiquement acceptables.

3. Agent contenant un composé de formule I selon la revendication 1.

4. Agent contenant deux ou plus de deux composés de formule I selon la revendication 1.

5. Agent selon la revendication 3 ou 4, caractérisé en ce qu'il contient un véhicule pharmaceutiquement acceptable et éventuellement d'autres additifs, tels que des stabilisants.

6. Composé selon la revendication 1 ou agent selon l'une des revendications 3 à 5, à utiliser en tant que médicament.

7. Composé ou produit selon la revendication 6, à utiliser en tant qu'inhibiteur de la secrétion d'acidité gastrique.

8. Composé de formule II, dans lequel $R^1$ à $R^5$, $Y^1$, $Y^2$ et Z sont tels que définis dans la revendication 2.

9. Procédé pour la préparation de composés de formule II selon la revendication 8, caractérisé en ce que

a) pour la préparation d'un composé de formule II, dans lequel $Y^1$ et $Y^2$ représentent une liaison et Z est un groupe séparable, on fait réagir un composé de formule IV

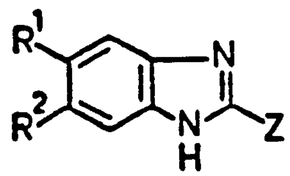
(IV)

dans laquelle $R^1$, $R^2$ sont tels que définis dans la revendication 1 et Z est tel que defini plus haut, avec un dérivé de picolyle substitué de formule V

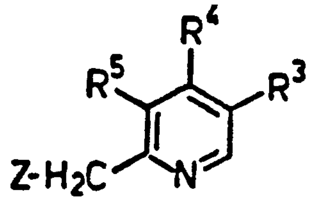
(V)

dans laquelle $R^3$ à $R^5$ sont tels que définis dans la revendication 1 et Z est tel que défini plus haut; ou

b) pour la préparation d'un composé de formule II, dans lequel $Y^1$ est un atome d'hydrogène et $Y^2 + Z =$ oxo, on fait réagir un composé de formule VI

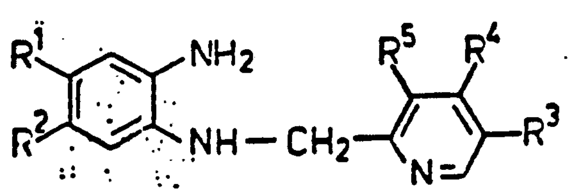
(VI)

dans laquelle $R^1$ à $R^5$ sont tels que définis plus haut, avec un dérivé d'acide carbonique.

10. Procédé pour la préparation d'un agent selon l'une des revendications 3 à 5, caractérisé en ce que l'on met des composés de formule I sous une forme pharmaceutique appropriée.

**Revendications** Pour l'Etat Contractant AT

1. Procédé pour la préparation de composés de formule I

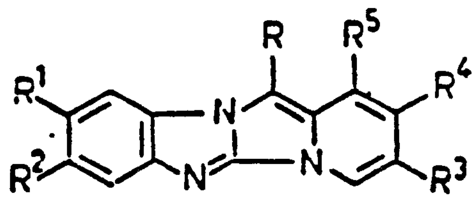
(I)

dans laquelle

$R^1$ et $R^2$ sont identiques ou différents, et représentent un atome d'hydrogène ou d'halogène ou un groupe alkyle en $C_1$-$C_6$, trifluorométhyle, méthoxycarbonyle, éthoxycarbonyle, alcoxy en $C_1$-$C_5$ ou alcanoyle en $C_1$-$C_4$;

$R^3$, $R^4$ et $R^5$ sont identiques ou différents, et représentent un atome d'hydrogène ou un groupe méthyle, méthoxy, éthoxy, méthoxyéthoxy ou éthoxyéthoxy; et

R représente un atome d'hydrogène ou un groupe $SR^6$, dans lequel $R^6$ représente l'électron non apparié du radical 12-sulfényle, un atome d'hydrogène, un groupe alkyle en $C_1$-$C_6$ qui peut éventuellement être substitué par un radical alcoxy en $C_1$-$C_4$ ou une ou plusieurs fois par un radical aryle en $C_6$-$C_{10}$, un groupe alcényle en $C_2$-$C_6$, alcinyle en $C_2$-$C_6$, alcanoyle en $C_1$-$C_7$, cycloalcanoyle en $C_5$-$C_7$, un groupe benzoyle qui peut éventuellement être substitué une ou plusieurs fois par un ou des radicaux alkyle en $C_1$-$C_7$, alcoxy en $C_1$-$C_5$, alcoxy($C_1$-$C_4$)-carbonyle, cyano, trifluorométhyle et/ou par des atomes d'halogène, un groupe furoyle, phénacyle ou un autre groupe protecteur usuel de fonction thiol;

et de leurs sels physiologiquement acceptables, caractérisé en ce que

a) pour la préparation de composés de formule I, dans lesquels R est un atome d'hydrogène,

$a_1$) on cyclise des composés de formule II

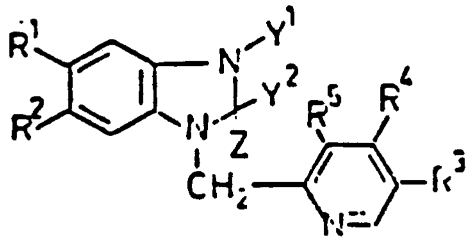

(II)

dans laquelle $R^1$ à $R^5$ sont tels que définis plus haut, et soit $Y^1 + Y^2$ représentent ensemble une liaison et Z représente un groupe séparable, soit

$Y^1$ représente un atome d'hydrogene et Y + Z forment ensemble un groupe oxo;

$a_2$) on cyclise des composés de formule IIIa

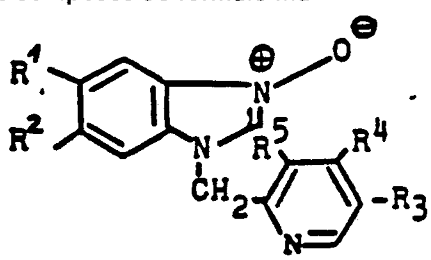

(IIIa)

dans laquelle $R^1$ à $R^5$ sont tels que définis plus haut;

b) pour la préparation de composés de formule I, dans lesquels R est le groupe sulfényle,

$b_1$) on traite par un acide un 2-benzimidazole-2'-pyridyl-sulfoxyde de formule III

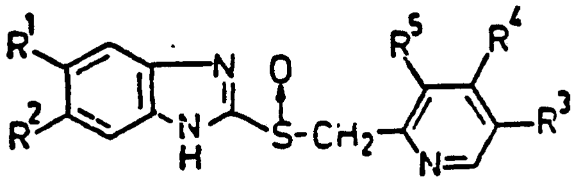

(III)

dans laquelle $R^1$ à $R^5$ sont tels que définis plus haut, ce par quoi, dans le cas où $R^1 \neq R^2$, il peut se former des mélanges de composés isomères de formule I à isomérie de position; ou

$b_2$) on fait réagir avec du soufre élémentaire ou avec $S_2Cl_2$, dans un solvant organique, des composés de formule I dans lesquels $R^1$ à $R^5$ sont tels que définis plus haut et R représente un atome d'hydrogène; ou

c) pour la préparation de composés de formule I, dans lesquels R est un atome d'hydrogène ou le groupe sulfényle, on réduit des composés de formule I dans lesquels $R^1$ à $R^5$ sont tels que définis plus haut, et R représente le groupe sulfényle, pour aboutir aux composés de formule I dans lesquels $R^6$ est un atome d'hydrogène, et si on le désire, on fait réagir ceux-ci avec des composés de formule $R^6X$, dans lesquels $R^6$ a la signification donnée plus haut, à l'exception d'un atome d'hydrogène, et X est un groupe séparable;

et éventuellement on convertit les composés obtenus selon $a_1$) à c) en leurs sels physiologiquement acceptables.

2. Procédé pour la préparation d'un produit contenant un composé de formule I selon la revendication 1, caractérisé en ce que l'on met celui-ci sous une forme pharmaceutique approprié.

3. Procédé selon la revendication 2, caractérisé en ce que l'on prépare un produit à partir de deux ou plus de deux composés de formule I.

4. Procédé selon la revendication 2 ou 3, caractérisé en ce que l'on prépare un produit qui contient un véhicule pharmaceutiquement acceptable et éventuellement d'autres additifs, tels que des stabilisants.

5. Procédé selon la revendication 1, pour la préparation d'un composé à utiliser en tant que médicament.

6. Procédé selon la revendication 1, pour la préparation d'un composé à utiliser en tant qu'inhibiteur de la secrétion d'acidité gastrique.

7. Procédé pour la préparation de composés de formule II, dans lesquels $R^1$ à $R^5$, $Y^1$, $Y^2$ et Z sont tels que définis dans la revendication 1, caractérisé en ce que

a) pour la préparation d'un composé de formule II, dans lequel $Y^1$ et $Y^2$ représentent une liaison et Z est un groupe séparable, on fait réagir un composé de formule IV

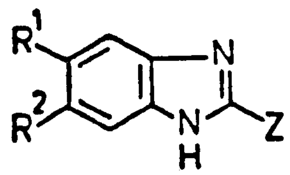

(IV)

dans laquelle $R^1$, $R^2$ sont tels que définis dans la revendication 1 et Z est tel que défini plus haut, avec un dérivé de picolyle substitué de formule V

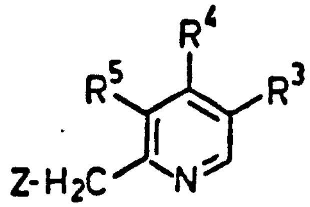

(V)

dans laquelle $R^3$ à $R^5$ sont tels que définis dans la revendication 1 et Z est tel que défini plus haut; ou

b) pour la préparation d'un composé de formule II, dans lequel $Y^1$ est un atome d'hydrogène et $Y^2 + Z =$ oxo, on fait réagir un composé de formule VI

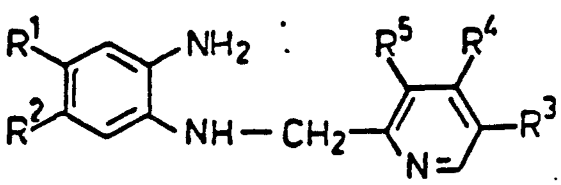

(VI)

dans laquelle $R^1$ à $R^5$ sont tels que définis plus haut, avec un dérivé d'acide carbonique.